# EUROPÄISCHE PATENTANMELDUNG

(11) **EP 4 265 734 A1**
(43) Veröffentlichungstag der Anmeldung: **25.10.2023**
(21) Anmeldenummer: 22191150.6
(22) Anmeldetag: 19.08.2022
(51) Int. Cl.: C12Q 1/6818, C12Q 1/6823

(54) **NUKLEINSÄURENACHWEIS IN EINER PCR MITTELS EINES ZIELSEQUENZ-UNSPEZIFISCHEM MODULARER REPORTERKOMPLEXES**

(30) Priorität: 22.04.2022 EP 22169463
(71) Anmelder: Hahn-Schickard-Gesellschaft für angewandte Forschung e.V., 78052 Villingen-Schwenningen (DE); Albert-Ludwigs-Universität Freiburg, 79098 Freiburg (DE)
(72) Erfinder: von Stetten, Felix, 79100 Freiburg (DE); Härpfer, Tamara, 79379 Müllheim (DE); Lehnert, Michael, 79098 Freiburg (DE); Trotter, Martin, 79232 March (DE); Borst, Nadine, 78052 Villingen-Schwenningen (DE); Becherer, Lisa, 77933 Lahr-Sulz (DE); Gmoser, Helena, 79252 Stegen (DE)
(74) Vertreter: Hertin und Partner Rechts- und Patentanwälte PartG mbB

(57) **Zusammenfassung**

Die Erfindung betrifft ein Verfahren zum Nachweis mindestens einer Ziel-Nukleinsäuresequenz mittels Mediatorsonde und mindestens einem Zielsequenz-unspezifischen modularen Reporter-Komplexes, wobei die freigesetzte Mediatorsequenz an eine Mediator-Bindestelle des Zielsequenz-unspezifischen modularen Reporter-Komplexes bindet und verlängert wird. Es wird eine Signaländerung initiiert und detektiert. Die Erfindung betrifft außerdem ein Kit zur Durchführung dieses Verfahrens.

## Beschreibung

### Technischer Hintergrund

Bei quantitativen Nachweisreaktionen von PCR-Produkten wie der Real-Time PCR (qPCR) oder der digitalen PCR (dPCR) werden Zielsequenzen entweder durch in die DNA interkalierende Farbstoffe, welche jedoch unspezifisch an alle vorhandene DNA-Doppelstränge binden, nachgewiesen oder durch DNA-Sonden, welche lediglich eine spezifische DNA-Zielsequenz binden. Diese DNA-Sonden erzeugen direkt (z.B. TaqMan-Sonden) oder indirekt (Mediatorsonden in Kombination mit universellen Reportermolekülen) durch ihre Spaltung eine optische Signaländerung. Optische Detektoren detektieren dabei die während der Reaktion generierten Lichtemissionen außerhalb des Reaktionsgefäßes. Diese Nachweissysteme greifen dabei zumeist auf lichtabsorbierende- und emittierende Fluoreszenzmoleküle zurück. Diese Moleküle geben nach Anregung durch Lichtenergie einer bestimmten Wellenlänge Energie in Form von höheren Wellenlängen ab, welche über Detektoren detektiert werden können. Dabei wird zwischen Molekülen unterschieden, deren Lichtenergie in einem bestimmten Wellenlängenbereich detektiert werden soll (Fluoreszenzdonoroder Fluorophor) und Molekülen, die zur Abnahme der Fluoreszenzintensität eines Fluorophors bei räumlicher Nähe führt (Fluoreszenzakzeptor oder Quencher). Ändert sich die die räumliche Nähe zwischen Fluorophor und Quencher, kommt es entsprechend zu einer Änderung des Fluoreszenzsignales, wobei ein geringerer Abstand stets ein höheres Quenching bewirkt (FRET-Quenching oder Kontakt-Quenching). Der Einsatz von Zielsequenz-spezifischen DNA-Sonden in Kombination mit unterschiedlichen Fluoreszenzmarkierungen ermöglicht weiterhin den sensitiven Nachweis von mehreren DNA-Sequenzen in einer Reaktion (Multiplex-Nachweis). Hierbei werden zumeist mehrere DNA-Sonden oder Nachweismoleküle eingesetzt, welche unterschiedliche Fluorophor-Quencher-Kombinationen tragen, welche in Gegenwart einer bestimmten DNA-Sequenz jeweils Fluoreszenzsignal in einem bestimmten Wellenlängenbereich emittieren. Weiterentwicklungen dieses Multiplexing-Nachweises ermöglichen zudem ebenfalls den Nachweis mehrerer Zielsequenzen innerhalb eines Wellenlängenbereichs, indem die Fluoreszenzwerte quantitativ betrachtet werden oder mit weiteren Parametern (z.B. der Auslesetemperatur) kombiniert werden.

Zwei bekannte einteiligen Zielsequenz-spezifischen DNA-Sonden zur optischen Detektion sind Taqman-Sonden und Molecular Beacons (Tan et al. 2004; Li et al. 2008; Holland et al. 1991; Rodriguez et al. 2005). Diese binden Zielsequenz-spezifisch und werden während der PCR gespalten, wodurch ein Signal generiert wird. Nachteil dieser Sonden ist die Abhängigkeit von der Zielgensequenz, womit einhergeht, dass nur spezielle Regionen einer DNA-Zielsequenz, welche bestimmten Anforderungen entsprechen müssen, verwendet werden können. Es muss beispielsweise auf Sondenlänge, Schmelztemperatur, Bindungsenthalpie, GC-Gehalt, Guanin-quenching und komplementäre Sequenzfragmente geachtet werden. Allerdings sind diese Sonden einfach zu synthetisieren, da in der Regel nur zwei endständige Markierungen benötigt werden. Neben diesen Zielsequenz-spezifischen DNA-Sonden gibt es auch fluorogene Nachweismoleküle, welche Zielsequenz-unspezifisch sind (z.B. Mediatorsonden und Universal Reporter). Diese haben den Nachteil, dass deren Synthese wesentlich anspruchsvoller ist, ermöglichen allerdings gänzlich neue Arten eines Sequenznachweises und besitzen eine hoch optimierte Fluoreszenzsignalgenerierung. Neben diesen Typen von einteiligen Nachweismolekülen gibt es ebenfalls zweiteilige, Zielsequenzspezifische Nachweissondensysteme (Light-Cycler Sonden). Hierbei wird ein Fluoreszenzsignal zwischen zwei benachbarten DNA-Sonden über deren Fluorophore mittels FRET in den langwelligeren Spektralbereich verschoben, sofern beide korrekt an die DNA-Zielsequenz binden und so den benötigten Abstand zueinander herstellen. Solche Systeme besitzen ebenfalls das Problem der Sequenzspezifität und bedürfen einer extremen Feinabstimmung im Design.

Molecular Beacons bestehen aus einem Oligonukleotid (Oligo) mit fünf bis sieben zueinander komplementäre Basen an den beiden Enden sowie einem endständigen Fluorophor bzw. Quencher (Tyagi und Kramer 1996). Bei Anlagerung der Enden durch Ausbildung eines Loops aufgrund der komplementären Basen werden die Fluoreszenzmoleküle in räumliche Nähe gebracht. Hierdurch wird die Fluoreszenz vom Donor auf den Akzeptor übertragen, wodurch die abgegebene Lichtwellenlänge durch Energieverlust in die langwelligere Wellenlänge des Akzeptors verändert oder gelöscht wird. Erst nach Bindung des Oligos an den komplementären Bereich der Zielsequenz und der damit einhergehenden Öffnung des Loops wird das Fluoreszenzquenching aufgehoben und eine Fluoreszenzsignalzunahme generiert. Dies ermöglicht die Detektion der Lichtenergie des Fluoreszenzdonors.

TaqMan-Sonden bestehen ebenfalls aus einem Oligonukleotid und zwei endständigen Fluoreszenzmolekülen (Heid et al. 1996). Allerdings bildet das Oligonukleotid keinen Loop aus, sondern die Fluoreszenz wird über räumliche Nähe am Oligonukleotid übertragen. Bei Anlagerung der Sonde an das Zielgen und Verlängerung eines Primers wird die Sonde durch die Exonukleaseaktivität der eingesetzten Polymerase abgebaut und somit Fluorophor von Quencher getrennt. Bei zu geringer Fluoreszenzlöschung durch eine besonders lange Sondensequenz und eine damit einhergehende große räumliche Trennung der Fluoreszenzmoleküle kann ein weiterer interner oder endständiger Quencher angebracht werden. Dies ist aber anspruchsvoller, da die genaue Länge der gespaltenen Sequenzen häufig unbekannt ist und es somit vorkommen kann, dass die gespaltene Sequenz mit Fluoreszenzdonor ebenfalls einen der internen Quencher enthält. In der digitalen PCR werden Taqman-Sondensysteme zudem für das sogenannte "Intensitäts-Multiplexing genutzt". Hierbei werden Taqman-Sonden mit identischer Fluoreszenzmarkierung aber unterschiedlichen DNA-Sequenzen eingesetzt, um mehrere Zielsequenzen in einer Multiplex- PCR zu differenzieren. Dies wird ermöglicht, indem die unterschiedlichen Typen von TaqMan-Sonden in einer unterschiedlichen Konzentration eingesetzt werden. Auch dieses Verfahren bedarf jedoch einer aufwendigen Feinabstimmung der Konzentrationen und ist zugleich meist nicht sehr präzise (Whale et al. 2016). Der in dieser Erfindung beschriebene modulare Reporterkomplex besitzt hierbei den Vorteil, dass unterschiedliche Fluoreszenzstärken bei gleichbleibenden Konzentrationen an Nachweismolekül-Komplexen eingestellt werden können, indem Komplexe aus mehreren Fluorophor- und Quencher-Markierten Oligonukleotiden ausgebildet werden. Dies bedarf weniger Feinabstimmung und ermöglicht eine präzisere Signaleinstellung.

Neben der direkten optischen Signalgenerierung durch eine fluoreszenzmarkierte DNA-Sonde gibt es ebenfalls Systeme, bei denen die Detektion über eine DNA-Sonde geschieht, welche keine Fluoreszenzmarkierung tragen. Diese binden ebenfalls Zielsequenz-spezifisch und werden durch die Exonuklease-Aktivität einer Polymerase gespalten. Im Gegensatz zu den bisher beschriebenen Sondentypen wird bei dieser Spaltung noch kein Fluoreszenzsignal generiert, sondern die Signalgenerierung an einem zweiten Molekül initiiert, welches selbst Zielsequenz-unabhängig ist. Dies wurde sowohl in der PCR als auch in der LAMP bereits gezeigt (Faltin et al. 2012; Faltin et al. 2013).

Eines dieser Verfahren wird als Mediatorsonden-PCR bezeichnet, welche 2012 durch die Universität Freiburg zum Patent angemeldet wurde. In diesem System werden in der PCR zwei Oligonukleotide statt nur einem zum Nachweis eingesetzt. Diese werden als Mediatorsonde (Mediator Probe / MP) und als Universeller Reporter (UR) bezeichnet. Bei der PCR Vervielfältigung der Zielsequenz bindet nur ein Teil der einer Mediatorsonde sequenzspezifisch und wird durch die Exonukleaseaktivität der Polymerase gespalten. Hierbei wird ein zweiter Teil der Mediatorsonde, der Mediator, welcher zuvor nicht an die Sequenz gebunden hat, abgetrennt. Ist keine Zielsequenz vorhanden, bleibt die Mediatorsonde intakt. Nach Abtrennung des Mediators wird dieser wiederum an einen Universal Reporter binden. Ein Universal Reporter ist Zielsequenz-unabhängig und besitzt neben der Mediatorbindestelle ebenfalls eine Fluorophor- und Quencher-Modifikation sowie eine Konformation, welche beide Modifikationen in eine räumliche Nähe zueinander bringt. Durch Verlängerung des Mediators am Universal Reporter wird diese räumliche Nähe, z.B. durch Abspaltung des Fluorophores, aufgehoben; wodurch ein Fluoreszenzsignal einer bestimmten Wellenlänge entsteht. Dieses Verfahren hat den Vorteil, dass es zu einer Trennung von Sonden-Anbindung an die DNA-Sequenz und Fluoreszenzsignalgenerierung kommt. Hierdurch können klare Richtlinien zum Mediatorsonden-Design aufgesetzt werden, universelle Reporter einmalig sehr weit optimiert können und dann für mehrere Sequenzen eingesetzt werden und zudem besteht mit diesem zweiteiligen Prozess eine doppelte Kontrolle, was die Signalgenerierung sehr spezifisch macht (Lehnert et al. 2018; Wadle et al. 2016, Schlenker et al 2021). Ein Nachteil dieser Technologie ist jedoch, dass entsprechende universal Reporter sehr aufwendig und teuer in ihrer Synthese sind, da ein Teil dieser Modifikationen intern in der DNA-Sequenz erfolgen müssen. Dies macht eine Weiterentwicklung dieser Moleküle hinsichtlich der Verleihung neuer Eigenschaften sehr aufwendig und schwierig. Zudem bedarf es noch weiterer Schutzgruppen an einem universellen Reporter.

Ein weiterer Mangel dieser Technologe ist überdies, dass sich je nach Gerät die Fluoreszenzbereiche eines Farbkanals unterscheiden, sodass für jedes Gerät eine eigene Fluorophor-Quencher-Optimierung durchgeführt werden muss. Diese Optimierung ist sehr aufwendig und kostenintensiv, da für jede Kombination ein neuer universeller Reporter synthetisiert werden muss. Zudem ist die Struktur des Oligonukleotids eingeschränkt, sodass nur wenige Möglichkeiten zum Anbringen von weiteren Markierungen für stärkere Signale oder Multiplexvariationen umsetzbar sind. Dies schränkt die Flexibilität des Reporters hinsichtlich des Testens verschiedener Markierungen stark ein. Der Nachteil dieses Systems ist somit die sehr teure Herstellung der universellen Reporter aufgrund dessen mehrfachen Modifikation sowie die unflexible Konstruktion.

Im gleichen Zeitraum wurde eine Technologie der Seegene Inc. entwickelt, welches ebenfalls auf einer Trennung von DNA-Sequenz Detektion und Signalgenerierung über zwei Nachweismoleküle in der real-time PCR beruht. Hierbei bindet eine unmarkierte PTO-Sonde an eine DNA-Zielsequenz, wird gespalten und setzt ein Fragment frei, welches anschießend mit einem fluoreszenzmarkiertem Zielsequenz-unspezifischen Nachweismolekül (CTO-Molekül) einen verlängerten Duplex bildet. Hierbei wird dieser Prozess dahingehend genutzt, um die Signalgenerierung über unterschiedliche Längen dieser Nachweismoleküle zu beeinflussen. Dies ermöglicht z.B. eine Differenzierung mehrerer Zielsequenzen im selben Detektionskanal, indem man bei definierten, vorab festgelegten Temperaturen das Signal ausliest. Ähnlich wie ein universeller Reporter ist auch eine CTO ein einzelnes Molekül, welches entsprechende Anforderungen an die Synthese stellt.

Ein weiteres Patent nutzt ebenfalls das Auslesen bei verschiedenen Temperaturen in einem Ansatz und kann entsprechend als Weiterentwicklung der oben genannten Technologie von Seegene interpretiert werden (PCT / CN2018 / 084794). Auch hierbei wird ein verlängertes Reportermolekül nach der Detektion aufgeschmolzen. Im Gegensatz zum vorherigen Patent kann hierbei ein Reportermolekül zur Differenzierung von verschiedenen Zielsequenzen genutzt werden.

Das Patent WO2013079307A1 Bifunktionale Oligonukleotidsonde zur universellen Echtzeit Multianalytdetektion beansprucht das System der Mediator-Sonden-Technologie. In diesem System wird eine Mediatorsonde aktiviert, indem ein Primer (auxiliary molecule 1) mittels einer Polymerase (auxiliary molecule 2) an der Zielsequenz (target molecule) verlängert wird. Aufgrund der Exonukleaseaktivität der Polymerase wird die Mediatorsonde gespalten und kann anschließend an den Universal Reporter (UR) binden (mediator hybridsation sequence). Hier fungiert er nach seiner Spaltung als Primer. Hierdurch wird er durch die Polymerase verlängert und trennt somit Fluorophor und Quencher am Universal Reporter.

Im Patent WO2018114674A1 zur schleifenvermittelten isothermalen Amplifikationsmethode mit Mediator-displacement Sonden (MD LAMP) wird ein universeller Reporter mit mindestens einem Oligonukleotid und mindestens einem Fluorophor und Quencher für eine LAMP-Reaktion beansprucht. Bisher wurde davon ausgegangen, dass dies nurfunktioniert, da eine LAMP im Gegensatz zu einer PCR kontinuierlich eine einheitliche Temperatur besitzt, wodurch sich schnell ein Gleichgewicht einstellt und beibehalten wird, was es einzelnen Molekülen ermöglicht dauerhaft aneinander zu binden und so im initialen Zustand kein Signal zu erzeugen.

Im allgemeinen Stand von Wissenschaft und Technik werden in optischen Nachweisreaktionen verschiedene Wellenlängen verwendet, um einzelne Zielsequenzen in bestimmten Lichtbereichen, den sogenannten Detektionskanälen, in einer Reaktion unterscheiden zu können. Dies schränkt das Multiplexen ein, da je Kanal nur eine Zielsequenz nachgewiesen werden kann. Somit ist der Multiplexgrad in den meisten kommerziellen Geräten auf fünf oder sechs Kanäle beschränkt.

Ein alternativer Ansatz zum Multiplexen stellen Patente mit Detektionssystemen dar, welche ebenfalls auf einer Trennung von Signalgenerierung und Detektion beruhen, wie die Patentschrift US20200087718A1 von Seegene zur Signalmolekül-basierten Detektion mittels Schmelzkurvenanalyse. Dieses Patent beansprucht die Möglichkeit zur Erhöhung des Multiplexgrades durch die Nutzung verschiedener Längen des Zielsequenz-unspezifischen Signalmoleküls, um unterschiedliche Schmelztemperaturen zu generieren. Eine weitere Teilanmeldung (EP2708608) nutzt gegenüber dem ursprünglichen Patent nur noch die Signalauslese bei vorab definierten Temperaturen, da die entsprechenden einteiligen Signalmoleküle unterschiedlicher Sequenzlängen ebenfalls unterschiedliches Fluoreszenzverhalten bei definierten Auslesetemperaturen zeigen.

Der in dieser Erfindung beschriebene modulare Reporter-Komplex hat den Vorteil, dass in bestimmen Ausführungsformen in einem Detektionskanal mehrere Zielsequenzen Temperatur-unabhängig nachgewiesen werden können. Hierzu dienen mehrere Fluorophore oder Quencher an einem Signalinitiations- oder Basisstrang, welche für unterschiedliche Fluoreszenzstärken sorgen und Fluoreszenzsignale so innerhalb desselben Kanals zuordbar machen. Dadurch können detektierte Zielsequenzen aufgrund der erzeugten Fluoreszenzstärke unterschieden werden. Dies ermöglicht die simultane Detektion von mindestens zwei Zielsequenzen innerhalb eines Detektionskanals ohne komplexe Temperaturabhängige Ausleseschritte.

Ein weiteres Patent ist das nur in den USA erteilte Patent der Firma Biorad (US 9921154 B2). Dieses beansprucht ebenfalls den Nachweis mehrerer Zielsequenzen in identischen Detektionskanälen in einer digitalen PCR, beschreibt aber lediglich sequenzspezifische mit Fluorophor und Quencher markierte Hydrolysesonden oder interkalierdend Farbstoffe für einen solchen Nachweis. Weitere Patente der Firma Biorad beschreiben ebenfalls die Unterscheidung verschiedener Zielsequenzen, z.T. in einem Kanal. Z.T. lassen sich diese nach aktuellen Verständnis aber nur dann zielführend einsetzen, wenn es zu einer deutlichen Überbelegung der Tropfen kommt.

### Aufgabe der Erfindung

PCR ist die Goldstandard Methode, um einzelne DNA-Sequenzen zu amplifizieren und so nachweisbar zu machen. Um PCR-Produkte (DNA oder cDNA im Fall von RNA) nachzuweisen und zu quantifizieren werden entweder interkalierende Farbstoffe oder DNA-Sonden genutzt. Interkalierende Farbstoffe binden jedoch unspezifisch an alle vorhandenen doppelsträngigen DNA-Moleküle, wodurch kein direkter Sequenzspezifischer Nachweis in der PCR möglich wird. Bei DNA-Sonden handelt es sich um signalerzeugende DNA-Sequenzen, welche komplementär zum jeweiligen Sequenzabschnitt eines PCR-Produktes sind und diese so spezifisch nachweisen und quantifizieren können. Insbesondere wird diese Methode in der real-time PCR und digitalen PCR genutzt. Aktuell verfügen DNA-Sonden entweder selbst über eine biochemische Modifikation, um in Gegenwart einer DNA-Zielsequenz während der PCR ein Signal zu erzeugen (z.B. Taqman-Sonden) oder aktivieren ein zweites Nachweismolekül, welches, Zielsequenz unabhängig, ein Signal generiert (z.B. Mediatorsonden in Kombination mit Zielsequenz-unspezifischen universellen Reportern).

Bei einer optischen Detektion z.B. über Fluoreszenz besitzen solche Nachweismoleküle, bzw. Nachweismolekül-Systeme gleichermaßen den Nachteil, dass sie in der Regel über mehrere biochemische Markierungen und Modifikationen auf einmal verfügen müssen (z.B. Fluorophor und Quencher im Falle einer fluorogenen Taqman-Sonde, Fluorophor, Quencher und 3'-Blockgruppe im Falle eines Zielsequenz-unspezifischen universellen Reporters), wodurch sie komplexer und teurer in ihrer Synthese sind und hierdurch unflexibel in ihrem Design werden. Dies ist insbesondere in der Entwicklung und Optimierung von DNA-Nachweisreaktionen ein großes Problem, da so nur wenige Systeme von Nachweismolekülen getestet werden können. Zudem sind die Möglichkeiten von Nachweismolekülen und somit Nachweismethoden hierdurch erheblich eingeschränkt, da nicht beliebig viele Modifikationen an einer einzelnen DNA-Sequenz vorgenommen werden können. Da während einer PCR die Bindungen zwischen DNA-Sequenzen mehrfach aufgetrennt und wieder geschlossen werden, wird im Stand der Technik jedoch davon ausgegangen, dass bei einem fluorogenen DNA-Nachweismolekül stets alle biochemischen Modifikationen an einer DNA-Sequenz gebunden sein müssen. Nur so, so die aktuelle Annahme im Stand der Technik, kann die initial benötigte räumliche Nähe zwischen Fluorophor und Quencher sichergestellt werden, um das Signal des Fluorophors durch den Quencher so lange zu unterdrücken, bis diese Moleküle im Rahmen des Nachweisprozesses räumlich getrennt werden. Die molekularen Prozesse, welche zu solchen Signalen führen sind zudem nicht immer einheitlich, da Sonden zur Signalgenerierung z.B. entweder gespalten werden oder aufklappen können. Dies jedoch führt zu einer inhomogenen Signalgenerierung, was zu unpräziseren Ergebnissen führt.

Dies zeigt den dringenden Bedarf an einem neuen Typus von Nachweisprozessen und Signalmolekülen auf, welche diese Probleme überwinden.

Die Erfinder haben festgestellt, dass diese Aufgabe durch eine neue Art eines modularen Nachweismolekülkomplexes gelöst werden sollte, welcher Zielsequenz-unspezifisch ist, flexibel im Design und leicht zu optimieren und hierbei dieselben Leistungsmerkmale besitzt, wie der aktuelle Stand der Technik sie für einteilige Nachweismoleküle beschreibt. Bisher scheiterte eine solche modulare Zusammensetzung insbesondere daran, dass diese auf kovalente chemische Bindungen verzichten, was sie nach aktuellen Annahmen des Stands der Technik ungeeignet für PCR-Anwendungen erscheinen lässt, da die Nachweismoleküle selbst während des thermische zyklischen Heizens getrennt würden.

### Zusammenfassung der Erfindung

Die erfindungsgemäße Aufgabe wird gelöst durch die Merkmale der unabhängigen Ansprüche. Vorteilhafte Ausgestaltungen der Erfindung sind in den abhängigen Ansprüchen beschrieben.

Daher bezieht sich die Erfindung in einem Aspekt auf ein Verfahren zum Nachweis mindestens einer Ziel-Nukleinsäuresequenz, umfassend die Schritte:
a. Bereitstellen mindestens eines Zielsequenz-unspezifischen modularen ReporterKomplexes, umfassend mindestens eine Markierung, sowie mindestens zwei Oligonukleotide, nämlich
   i. einen Basisstrang, umfassend
      1. mindestens eine Mediator-Bindestelle
      2. mindestens eine Signaloligo-Bindestelle
   ii. mindestens ein Signaloligo
   wobei die mindestens eine Signaloligo-Bindestelle des Basisstrangs und das mindestens eine Signaloligo miteinander hybridisieren, jedoch nicht kovalent verbunden sind und zusammen einen Signal-Komplex bilden,
b. Bereitstellen mindestens einer Mediatorsonde, wobei die Mediatorsonde ein Oligonukleotid mit mindestens einer Sondensequenz und mindestens einer Mediatorsequenz umfasst, wobei
   die mindestens eine Sondensequenz eine Affinität zu mindestens einer Ziel-Nukleinsäuresequenz aufweist, und die mindestens eine Mediatorsequenz eine Affinität zu mindestens einer Mediator-Bindestelle an dem Basisstrang des mindestens einen Zielsequenz-unspezifischen modularen Reporter-Komplexes aufweist,
c. PCR Amplifikation mindestens einer Nukleinsäuresequenz,
d. Binden einer Sondensequenz mindestens einer Mediatorsonde an die mindestens eine Ziel-Nukleinsäuresequenz,
e. Spaltung der Sondensequenz der an die mindestens eine Ziel-Nukleinsäuresequenz gebundene mindestens eine Mediatorsonde durch eine PCR Polymerase mit Nukleaseaktivität während der PCR Amplifikation, wobei die Mediatorsequenz freigesetzt wird,
f. Binden mindestens einer freigesetzten Mediatorsequenz an eine Mediator-Bindestelle des mindestens einen Zielsequenz-unspezifischen modularen ReporterKomplexes,
g. Verlängerung der Sequenz mindestens einer an eine Mediator-Bindestelle gebundenen Mediatorsequenz durch eine PCR Polymerase, wobei die Bindung der miteinander hybridisierten mindestens einen Signaloligo-Bindestelle und des mindestens einen Signaloligos aufgebrochen wird, wodurch eine Signaländerung initiiert wird,
h. Detektion mindestens einer Signaländerung als Nachweis der mindestens einen Ziel-Nukleinsäuresequenz.

In einer bevorzugten Ausführungsform des erfindungsgemäßen Verfahrens umfasst die mindestens eine Markierung des Zielsequenz-unspezifischen modularen Reporter-Komplexes mindestens ein Fluorophor und/oder mindestens einen Quencher.

In Ausführungsformen dient das vorliegende Verfahren zum Nachweis von mindestens einer Nukleinsäuresequenz während einer PCR Reaktion, wobei während dieser Reaktion eine Mediatorsonde gespalten (durch eine Exonukleaseaktivität der Polymerase) wird. Das Spaltprodukt ist ein Mediator, welcher im Folgenden an einem Zielsequenz-unspezifischen modularen Reporterkomplex bindet und hier über eine Folgereaktion eine Signaländerung initiiert, welche dem Nachweis der DNA-Sequenz dient. Der Zielsequenz-unspezifische modularer Reporter-Komplex besteht in dieser Ausführungsform vorzugsweise aus mindestens zwei Oligonukleotiden, welche nicht kovalent verbunden sind, wobei mindestens ein Oligonukleotid dieses Komplexes mindestens eine Markierung besitzt, welche eine Signaländerung initiiert und welche vorzugsweise mindestens ein Fluorophor und mindestens einen Quencher umfasst. In dieser Ausführungsform besitzt ein Oligonukleotid des Zielsequenz-unspezifischen modularen Reporter-Komplex (Basisstrang) vorzugsweise mindestens eine Bindestelle für mindestens eine Mediatorsequenz (hierein auch als Rezeptor bezeichnet) und mindestens eine Bindestelle für ein Signaloligo. In dieser Ausführungsform ist das zweite Oligonukleotid des Komplexes das Signaloligo, welches an den Basisstrang bindet (und so einen Signal-komplex bildet). Solange die Mediatorsonde ungespalten ist, bleibt der Zielsequenz-unspezifische modulare Reporter-Komplex während des Detektions- oder Ausleseprozesses in seinem Grundzustand. Durch eine im Rahmen der PCR erfolgenden Verlängerung des Mediator-Oligonukleotids entlang des Basisstrangs, wird der Zielsequenz-unspezifische modulare Reporter-Komplex am Signalkomplex in einer Weise aufgebrochen, dass die Markierung oder Markierungen am Basisstrang und/oder am Signaloligo voneinander separiert werden, wodurch eine Änderung des Signals zum Grundzustand und somit eine Signaländerung initiiert wird, welches dem Nachweis der DNA-Zielsequenz dient.

In manchen Ausführungsformen können die Schritte d - h des erfindungsgemäßen Verfahrenswährend der PCR Amplifikation kontinuierlich in jedem Zyklus ablaufen, dieses ist vorzugweise der Fall, wenn die PCR Amplifikation eine real-time PCR oder qPCR ist. In anderen Worten, in Ausführungsformen des erfindungsgemäßen Verfahrens werden die Schritte d - h während der PCR Amplifikation in jedem PCR-Zyklus wiederholt, wobei die PCR Amplifikation eine real-time PCR oder qPCR ist.

In anderen Ausführungsformen können die Schritte d - g des erfindungsgemäßen Verfahrens während der PCR Amplifikation ablaufen, wobei der Schritt h anschließend erfolgt, dieses ist vorzugweise der Fall, wenn die PCR Amplifikation eine digitale PCR oder Endpunktanalyse ist. In anderen Worten, in Ausführungsformen des erfindungsgemäßen Verfahrens werden die Schritte d - g während der PCR Amplifikation in jedem PCR-Zyklus wiederholt, und anschließend erfolgt Schritt h, wobei die PCR Amplifikation eine digitale PCR oder Endpunktanalyse ist. Im Falle einer digitalen PCR und/oder Endpunktanalyse erfolgt nämlich der Detektionsschritt h des erfindungsgemäßen Verfahrens vorzugsweise separat, nachfolgend auf die PCR-Amplifikationsreaktion.

Das erfindungsgemäße modulare Reporter-System der Zielsequenz-unspezifischen Detektion grenzt sich durch den flexiblen Einsatz verschiedener Oligonukleotide mit unterschiedlichen Markierungen von den bisher bekannten Detektionssystemen ab und besitzt dabei überraschenderweise dieselben Leistungskennzahlen in einer PCR, wie aktuelle einteilige Nachweismoleküle. Vor allem der modulare Aufbau erbringt diverse Vorteile wie das flexibel anpassbare Design an bestimmte Gerätebedingungen, die Einheitlichkeit der Signalgenerierungsreaktion zwischen verschiedenen Detektions-Methoden oder die günstige, einfache Herstellung sowie gänzlich neue multiplex Nachweismethoden. Das System und seine Funktionalität wurde bisher weder in einem Patent noch in der Literatur zur Nukleinsäure-Detektion in einer PCR beschrieben.

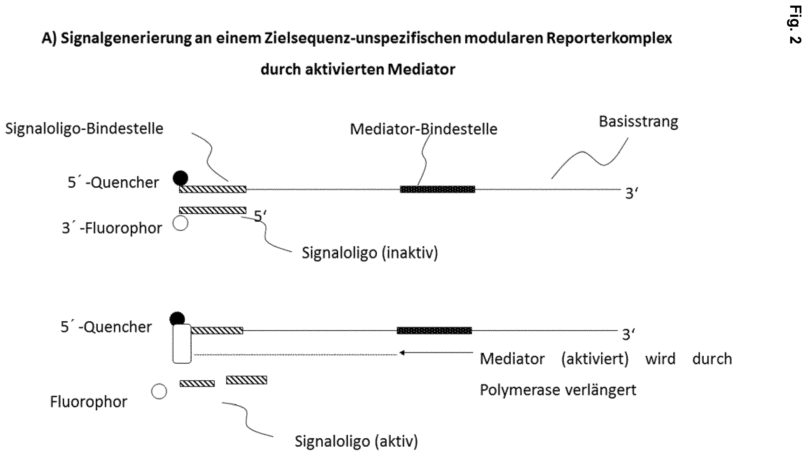

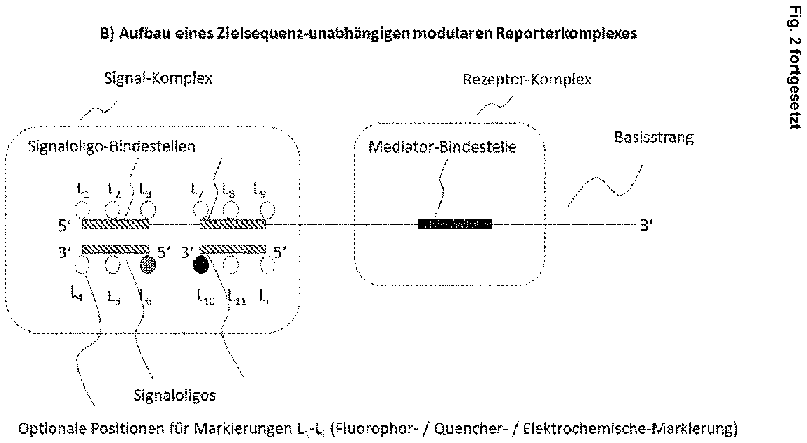

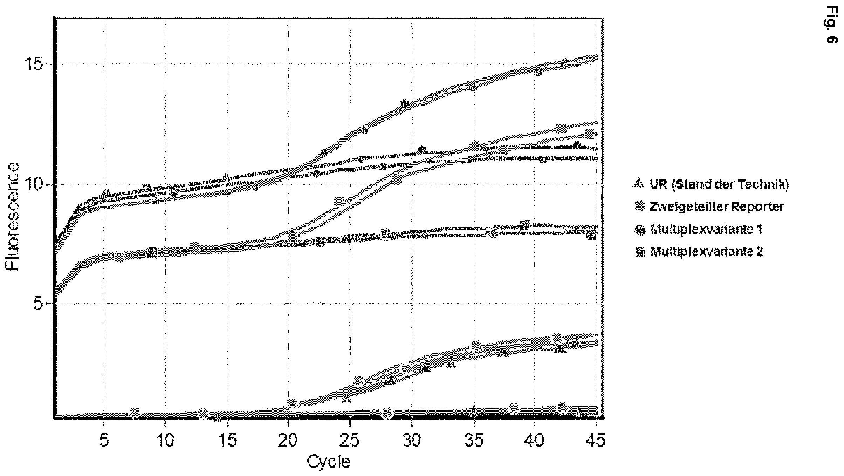

Den Kern der Erfindung bildet der modular aufgebaute Zielsequenz-unabhängige modulare Reporterkomplex (siehe Figur 2A für eine beispielhafte Ausführungsform) aus nicht kovalent miteinander verbundenen Oligonukleotiden. Dieses System besitzt alle Vorteile der verschiedenen Zielsequenz-spezifischen Detektionssysteme sowie Zielsequenz-unspezifischer Nachweismoleküle und kombiniert sie mit einer erhöhten Flexibilität im Design solcher Nachweismoleküle, welche zu gänzlich neuen Nachweismethoden führen. Überraschenderweise kann das System in einer PCR mit zyklischer Temperaturveränderung, und der damit einhergehenden Aufschmelzung der DNA-Stränge in jedem Zyklus, ohne Probleme eingesetzt werden. Die Ergebnisse der vorliegenden Beispiele, welche auch in den Figuren 6-8 dargestellt sind, liefern einen Beweis, dass das erfindungsgemäße modulare System Universal Reporter (UR) des Standes der Technik sogar in deren Funktionalität übertrifft. Hierbei kann die deutlich günstigere Herstellung bei mindestens genauso guten Leistungsparameters erzielt werden. Ein weiterer Vorteil des Systems ist die Möglichkeit zur flexiblen Anpassung der erfindungsgemäßen universellen Sonde. Es können sowohl verschiedene Fluorophore und Quencher günstig miteinander kombiniert und untersucht werden als auch Multiplexreaktionen erweitert werden. Mittels verschiedenen Markierungen können verschiedene Sonden in einem Fluoreszenzkanal unterschieden werden. Somit stellt die Erfindung ein dynamisches, günstiges und universelles Detektionssystem zur Verfügung.

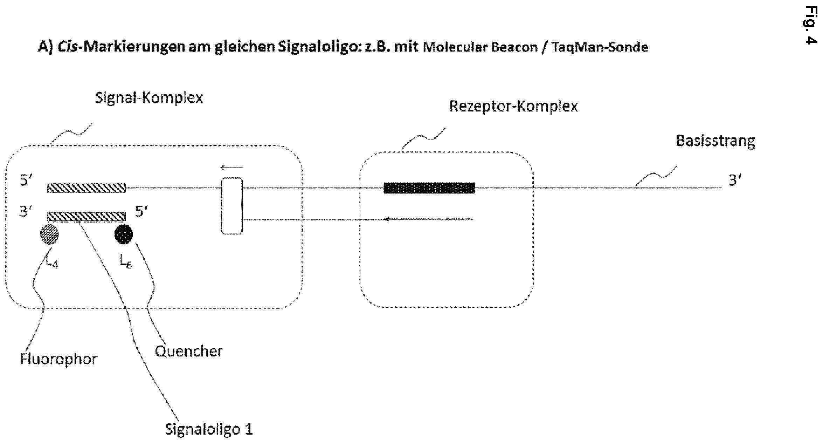

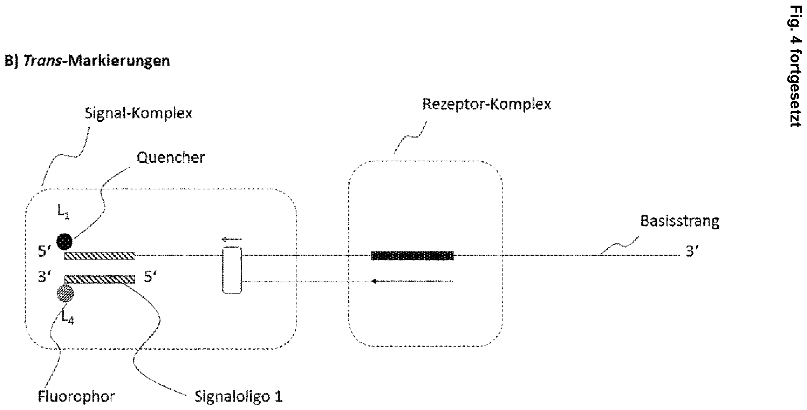

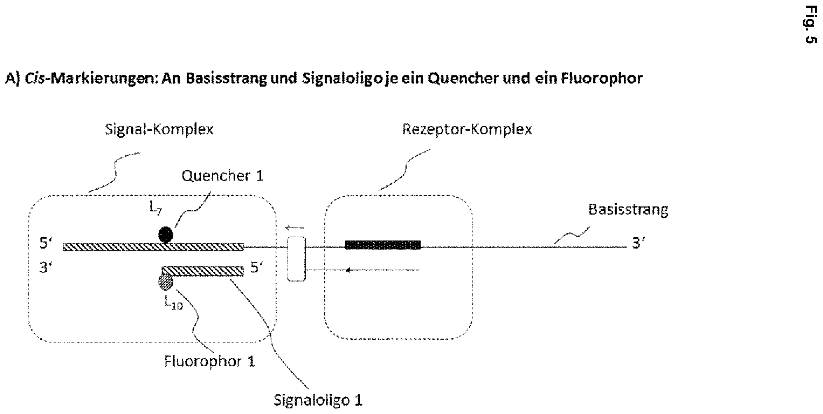

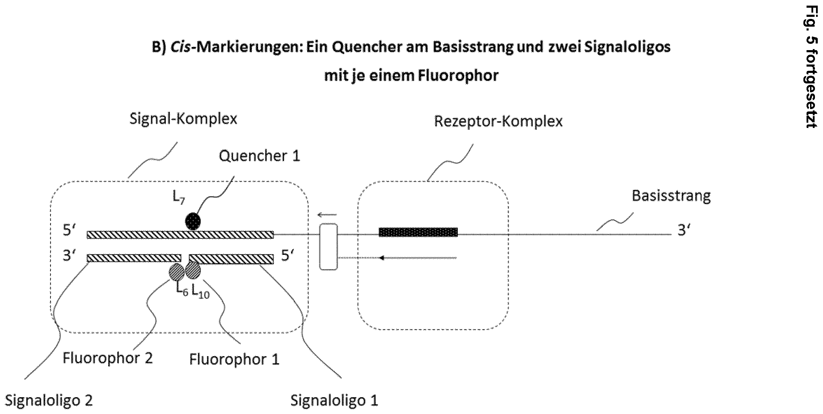

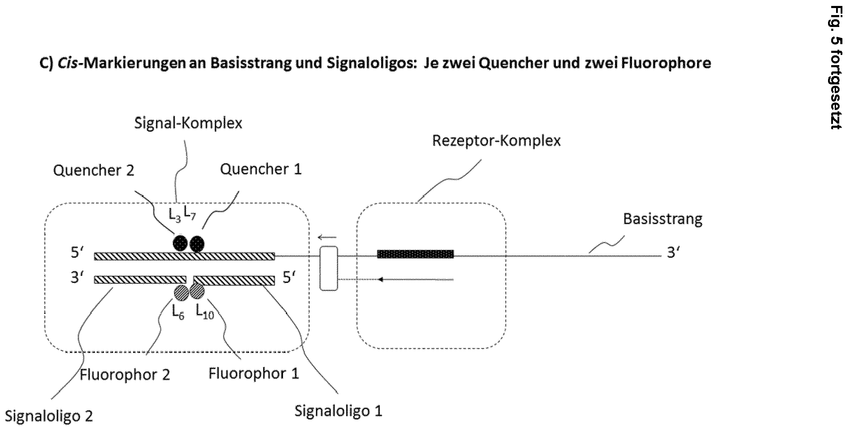

Das modulare System des erfindungsgemäßen Zielsequenz-unabhängigen modularen Reporter-komplexes besteht im Grundgerüst aus einem Basisstrang. Dieser besitzt vorzugsweise mindestens eine Bindestelle für einen Mediator (Rezeptor-Komplex) und mindestens einer Bindestelle für ein Signalinitiationsoligonukleotid (oder kurz "Signaloligo") und formt somit einen Signal-Komplex (Beispiele für Ausführungsformen umfassend einen Signalkomplex und einen Rezeptorkomplex finden sich z.B. in den Figuren 2, 4 und 5). Der Basisstrang und mindestens ein Signaloligo bilden gemeinsam einen Komplex aus (siehe z.B. Figur 2B für ein beispielhafte Ausführungsform). Vorzugsweise besitzt mindestens einer dieser Stränge hierbei eine Markierung, welche eine strukturelle Änderung dieses Komplexes nachweisen kann. Diese strukturelle Änderung stellt vorzugsweise die Trennung, Verdrängung, bzw. die (Ab-)Spaltung, Loslösung oder den enzymatischen Verdau, des Signaloligos vom Basisstrang dar, was vorzugsweise zu einer Signaländerung führt. Der modulare Aufbau ergibt sich in Ausführungsformen aus mehreren möglichen Signalinitiationsoligonukleotiden (oder Signaloligonukleotiden, kurz "Signaloligos") sowie hierüber zusätzlichen Markierungen, welche sowohl an den Signaloligonukleotiden als auch am Basisstrang angebracht werden können (siehe z.B. Figur 2B, Markierungspositionen L₁ bis Lᵢ am Signal-Komplex). Durch verschiedene Möglichkeiten zur Anbringung von Cis- oder Trans-Markierungen können die Vorteile von Zielgen-abhängigen Systemen des Stands der Technik aufgegriffen werden. Verschiedene beispielhafte Ausführungsformen finden sich in den Figuren 4 und 5. Durch den modularen Aufbau kann eine weitaus höhere Anzahl an Markierungen je Komplex angebracht werden als es bei aktuellen einteiligen Nachweismolekülen möglich ist.

Somit umfasst in Ausführungsformen des erfindungsgemäßen Verfahrens der Basisstrang mindestens eine Signaloligo-Bindestelle, an welche zwei oder mehr Signaloligos hybridisiert sind, und wobei die zwei oder mehr Signaloligos und/oder der Basisstrang an der mindestens einen Signaloligo-Bindestelle eine oder mehrere Markierungen besitzen.

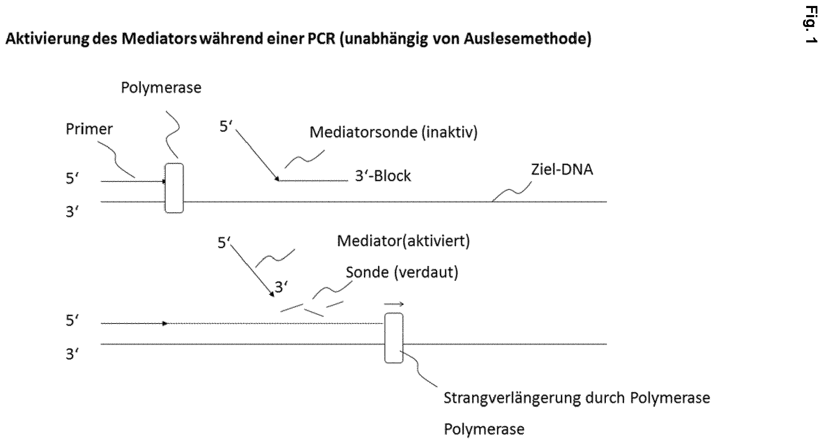

Aufgrund dieser Neuartigkeit des modularen Nachweiskomplexes ergeben sich neue Möglichkeiten für Nachweisreaktionen, welche auf einer Basisreaktion beruhen welche im Folgenden am Beispiel einer optischen Detektion beschrieben ist (siehe Figuren 1 und 2A für ein beispielhafte Ausführungsform):
In einer Ausführungsform bindet während einer PCR-Nachweisreaktion eine Mediatorsonde an die amplifizierte DNA-Zielsequenz. Eine Mediatorsonde ist vorzugsweise ein Oligonukleotid und besitzt einen sequenzspezifischen Sondenabschnitt, welcher an die Zielsequenz bindet und am 3'-geschützt ist, und einen Zielsequenz-unspezifischen Abschnitt, Mediator genannt, welcher bis auf ein Nukleotid welches Mediator und Sonde gemeinsam ist, nicht an die Zielsequenz bindet. Während der Primerverlängerung durch eine Polymerase mit Exonukleaseaktivität (z.B. im Rahmen einer Amplifikationsreaktion) wird der Mediator von der Sonde abgespalten wobei die gemeinsame Base am Mediator verbleibt (Figur 1). Der Mediator ist nun nicht mehr durch den Sondenabschnitt blockiert und kann nun an den Zielsequenz-unspezifischen-Reporter-Komplex binden und hier verlängert werden. Hierbei bindet der Mediator an den Rezeptorkomplex des Basisstrangs des Zielsequenz-unabhängigen modularen Reporterkomplexes. Anschließend wird der Mediator durch die Polymerase entlang des Basisstranges verlängert, wobei der Signalkomplex in einer Weise aufgebrochen wird, dass einzelne Bestandteile und/ oder Moleküle dieses Komplexes abgespalten werden und hierdurch eine Signaländerung gegenüber dem Urzustand initiiert wird (Figur 2A).

In bevorzugten Ausführungsformen des erfindungsgemäßen Verfahrens wird keine Fluoreszenzsignaländerung durch den mindestens einen Fluorophor generiert, wenn das mindestens eine Signaloligo mit der mindestens einen Signaloligo-Bindestelle des Basisstranges hybridisiert ist, wobei entweder der mindestens eine Quencher an der mindestens einen Signaloligo-Bindestelle des Basisstranges lokalisiert ist und der mindestens eine Fluorophor an dem mindestens einen Signaloligo oder umgekehrt, und wobei in Schritt g mindestens ein Fluorophor und mindestens ein Quencher separiert werden, wodurch eine Signaländerung initiiert wird.

Im Kontext der Erfindung beschreibt eine Fluoreszenzsignaländerung vorzugsweise eine signifikante, differenzierbare und/oder charakteristische Änderung des Fluoreszenzsignals, welche sich deutlich von potentiellen Basis- oder Hintergrundsignalen bzw. Grund- oder Hintergrundrauschen abgrenzt bzw. unterscheidet. Daher beschreibt eine Fluoreszenzsignaländerung im Kontext der Erfindung vorzugsweise eine signifikante, differenzierbare und/oder charakteristische Änderung des Fluoreszenzsignals, und kein Fluoreszenz-Basis- oder Hintergrundsignal bzw. Grund- oder Hintergrundrauschen.

In Ausführungsformen umfasst zudem die mindestens eine Markierung mindestens ein Fluorophor und mindestens einen Quencher, wobei sowohl der mindestens eine Quencher als auch der mindestens eine Fluorophor auf dem mindestens einen Signaloligo lokalisiert sind, und wobei in Schritt g. (Verlängerung der Sequenz mindestens einer an eine Mediator-Bindestelle gebundenen Mediatorsequenz durch eine PCR Polymerase) das mindestens eine Signaloligo durch die PCR Polymerase abgespalten wird, wodurch der mindestens eine Fluorophor und der mindestens eine Quencher separiert werden, wodurch eine Signaländerung initiiert wird. In Ausführungsformen kann die Abspaltung des Signaloligos von der Signaloligo-Bindestelle des Basisstrangs durch eine PCR-Polymerase entweder durch einen enzymatischen Verdau oder Spaltung des Signaloligos durch die Polymerase (z.B. durch Exonukleaseaktivität der Polymerase) oder durch einen anderen Mechanismus erfolgen, z.B. indem das Signaloligo durch die Polymerase vom Basisstrang abgelöst, davon getrennt oder verdrängt wird.

Der erfindungsgemäße Prozess zum Nachweis von DNA-Sequenzen mittels PCR in Kombination mit einer optischen Auslese umfasst ein neuartiges System aus einzelnen Oligonukleotiden, vorzugsweise DNA-Oligonukleotiden. Diese bilden einen solchen Zielsequenz-unabhängigen modularen Reporterkomplex ohne kovalente Bindungen aus. Dieser Zielsequenz-unabhängige modulare Reporterkomplex besitzt dabei überraschenderweise alle Vorteile und Leistungskennzahlen einteiliger Zielsequenz-abhängiger DNA-Sonden oder einteiligen Zielsequenz-unabhängigen Nachweismoleküle.

Der Zielsequenz-unabhängige modulare Reporterkomplex besteht vorzugsweise aus mindestens zwei DNA-Sequenzen, welche spezifisch aneinander binden und chemische Modifikationen tragen, welche die Signalgenerierung während einer PCR Reaktion (z.B. DNA-Amplifikation) initiieren. In manchen Ausführungsformen können durch die Verwendung von unterschiedlichen Zielsequenz-unabhängigen Reporter-Komplexen mit einer unterschiedlichen Anzahl von Markierungen und/ oder unterschiedlichen Anzahl an Signaloligos unterschiedlich starke Signale generiert werden, welche die Aktivierung dieser Zielsequenz-unabhängigen Reporterkomplexen unterscheidbar machen. Somit können durch die Kombination mehrere oder verschiedener Markierungen (z.B. unterschiedliche Farbe und/oder Intensität) voneinander unterscheidbare Signale generiert werden. So kann zum Beispiel das Signal eines Signaloligos mit einer roten Markierung von dem Signal eines Signaloligos mit zwei oder drei roten Markierung anhand der Intensitätsunterschiede des erzeugten Signals, oder von dem Signal eines Signaloligos mit einer roten und einer grünen Markierung farblich unterschieden werden. Im Rahmen von Ausführungsformen der vorliegenden Erfindung sind nicht nur einzelne Markierungen, sondern auch unterschiedliche Kombinationen von verschiedenen Fluorophor-Farben und/oder der Anzahl an Fluorophoren (Signalintensität) vorgehsehen, welche jeweils ein Signal kodieren das für eine Zielsequenz spezifisch sind. Diese Kombinierbarkeit von Signalen ist vorteilhaft für die Detektion mehrerer Zielsequenzen zur gleiche Zeit (in der gleichen PCR Reaktion).

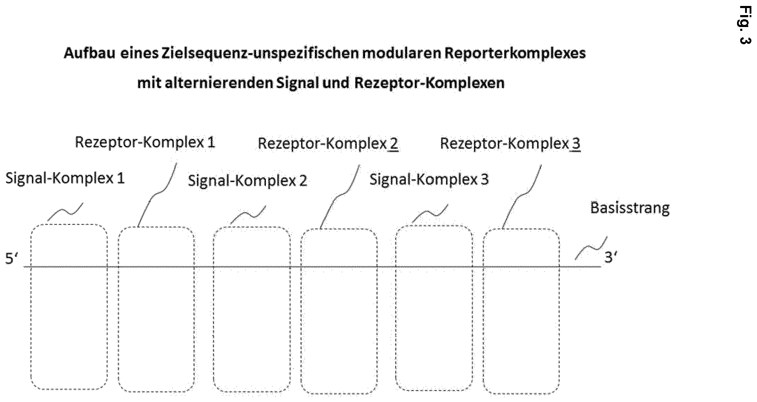

Multiplex-Nachweisreaktionen, also ein gleichzeitiger Nachweis verschiedener Zielsequenzen in einer Probe und in einer Reaktion, erfordern im bisherigem Stand der Technik entweder die Verfügbarkeit mehrerer optischer Kanäle, weitere Prozessschritte oder eine aufwendige Konzentrationsabstimmung der Reportermoleküle. Im Gegensatz ermöglicht der erfindungsgemäße Zielsequenz-unabhängige modulare Reporterkomplex eine kombinierte Detektion über verschiedene Kanäle, wozu ein Basisstrang mit mehr als einem Rezeptorkomplex verwendet werden kann. In diesen Ausführungsformen sind mehrere Rezeptorkomplexe (≥ 2) versetzt entlang des Basisstrangs angebracht, sodass sie unterschiedliche Signalkomplexe aktivieren können (siehe Figur 3 für ein Ausführungsbeispiel). Hierbei kann in verschiedenen Ausführungsformen ein Rezeptor-Komplex ein oder mehrere, vorzugsweise stromaufwärts (gen 5'-Ende) gelegene, Signal-Komplexe regulieren bzw. aktivieren.

Somit umfasst in Ausführungsformen des erfindungsgemäßen Verfahrens der Basisstrang mindestens eine Signaloligo-Bindestelle, an welche zwei oder mehr Signaloligos hybridisiert sind, und wobei die zwei oder mehr Signaloligos und/oder der Basisstrang an der mindestens einen Signaloligo-Bindestelle eine oder mehrere Markierungen besitzen.

In manchen Ausführungsformen umfasst der Basisstrang zwei oder mehr Signaloligo-Bindestellen, und wobei mindestens eines der Signaloligos, welche mit den zwei oder mehr Signaloligo-Bindestellen hybridisiert sind und/oder der Basisstrang an mindestens einer der zwei oder mehr Signaloligo-Bindestellen eine oder mehrere Markierungen besitzt.

In manchen Ausführungsformen ermöglicht der mindestens eine Zielsequenz-unspezifische modulare Reporter-Komplex den Nachweis von mindestens einer ersten und einer zweiten Ziel-Nukleinsäuresequenz, indem der Basisstrang,
mindestens eine erste und eine zweite Mediator-Bindestelle für mindestens eine erste und eine zweite Mediatorsequenz mindestens einer ersten und einer zweiten Mediatorsonde, umfasst, sowie mindestens eine erste und eine zweite Signaloligo-Bindestelle, an welche mindestens ein erstes und ein zweites Signaloligo hybridisiert ist, und
wobei die erste Mediatorsonde eine Sondensequenz mit einer Affinität zu einer ersten Ziel-Nukleinsäuresequenz und die zweite Mediatorsonde eine Sondensequenz mit einer Affinität zu einer zweiten Ziel-Nukleinsäuresequenz aufweist.

In manchen dieser Ausführungsformen weist der Basisstrang mindestens eine erste und eine zweite Markierung auf, wobei die Signaländerung durch die mindestens eine erste Markierung charakteristisch für die erste Ziel-Nukleinsäuresequenz ist, und die Signaländerung durch die mindestens eine zweite Markierung charakteristisch für die zweite Ziel-Nukleinsäuresequenz ist.

In manchen Ausführungsformen wird in Schritt a. mindestens ein erster und ein zweiter Zielsequenz-unspezifischer modularer Reporter-Komplex bereitgestellt, wobei der mindestens erste Zielsequenz-unspezifische modulare Reporter-Komplex den Nachweis mindestens einer ersten Ziel-Nukleinsäuresequenz und der mindestens zweite Zielsequenz-unspezifische modulare Reporter-Komplex den Nachweis einer zweiten Ziel-Nukleinsäuresequenz ermöglicht, wobei die Signaländerung durch die mindestens eine Markierung des mindestens ersten Zielsequenz-unspezifischen modularen Reporter-Komplexes charakteristisch für die erste Ziel-Nukleinsäuresequenz und die Signaländerung durch die mindestens eine Markierung des mindestens zweiten Zielsequenz-unspezifischen modularen Reporter-Komplexes charakteristisch für die zweite Ziel-Nukleinsäuresequenz ist.

Bevorzugt können durch unterschiedliche Farbkombinationen unterschiedliche verlängerte Mediatorsequenzen kodiert werden sodass unterschiedliche Zielsequenzen nachgewiesen werden können. Somit ist ein erfindungsgemäßer Zielsequenz-unabhängige modulare Reporterkomplex flexibel im Design.

Daher unterscheiden sich in manchen Ausführungsformen die für die mindestens erste und die für die mindestens zweite Ziel-Nukleinsäuresequenz charakteristischen Signaländerungen von einander durch ihre Farbe und/oder ihre Fluoreszenz- oder Signalstärke.

In Ausführungsformen können verschiedene Kodierungen von Fluoreszenzsignalen verwendet werden. In einer Ausführungsform umfasst ein Signal-Komplex mindestens zwei Signaloligos mit zwei Markierungen, wobei das gemeinsame Signal charakteristisch für eine Zielsequenz ist.

Somit sind in Ausführungsformen die Kombination mehrere Signaloligos mit jeweils mindestens einer Markierung möglich, um ein für eine gemeinsame Zielsequenz spezifisches Signal zu generieren. Durch die Kombination mehrerer gleich oder unterschiedlich farbiger Markierungen können Signale generiert werden, welche sich von anderen Signalen für andere Zielsequenzen durch ihre Farbe und/oder ihre Intensität unterscheiden. Durch die Kombination verschiedener Fluoreszenzfarben von Markierungen und optional zusätzlich durch deren Anzahl können so auch Mischfarben generiert werden.

In einer anderen Ausführungsform umfasst der Basisstrang mindestens zwei Signal-Komplexe mit unterschiedlichen Signaloligos und/oder unterschiedlichen Markierungen an den Signaloligos, wobei der Basisstrang einen einzelnen zu den mindestens zwei Signal-Komplexen korrespondierenden Rezeptorkomplex mit mindestens einer Mediator-Bindestelle umfasst, z.B. stromabwärts (gen 3'-Ende) der Signalkomplexe. In dieser Ausführungsform kann eine Zielsequenz durch zwei Farben kodiert werden.

Der erzielte Effekt dieser Ausführungsformen ist ein kolorimetrisch kodiertes Multiplexing in der digitalen PCR oder qPCR Nachweis- bzw. Amplifikationsreaktion, was den Nachweis von mehr (einer größeren Zahl von) Zielsequenzen ermöglicht, als dass Detektionskanäle verfügbar sind. Ein Beispiel für eine Farbkodierung von Fluorophoren wären die Signal-Kombinationen: Zielsequenz 1: rot-rot, Zielsequenz 2: rot-grün, Zielsequenz 3: grün-grün, Zielsequenz 4: grün-grün-rot.

Der Nachweis mehrerer Zielsequenzen innerhalb eines Wellenlängenbereichs kann durch eine quantitative Betrachtung der Fluoreszenzwerte erfolgen oder mit weiteren Parametern (z.B. der Auslesetemperatur) kombiniert werden. Die Signalgenerierung kann über unterschiedliche Längen der Signaloligos beeinflusst werden, sodass eine Differenzierung mehrerer Zielsequenzen im selben Detektionskanal erfolgen kann. Hierfür müssen vor der Analyse spezifische Auslesetemperaturen für die Signal-Detektion festgelegt werden. Je nach den gewählten Parametern wie der Länge des Signaloligos und/ oder den gewählten Fluorophoren ergibt sich so ein unterschiedliches Signal bei unterschiedlichen Temperaturen. Durch das iterative Auslesen des Fluoreszenzsignals bei verschiedenen Temperaturen lässt sich so bei bestimmten Temperaturbereichen eine spezifische Signaländerung erkennen, welche charakteristisch für die gewählten Fluoreszenzmodifikationen oder Längen der Signaloligos ist. Hierdurch kann darauf zurückgeschlossen werden, welcher modulare Reporterkomplex aktiviert wurde.

Daher umfasst in manchen Ausführungsformen die Detektion der Signaländerung eine Analyse der Signaländerung in Abhängigkeit der Detektions-Temperatur.

Die hier beschriebene erfindungsgemäße Methode besitzt gegenüber der in US 9921154 B2 beschriebenen Multiplexing-Methode den Vorteil, dass sich die erfindungsgemäße Methode auch ohne eine Mehrfachbelegung jedes Reaktionsraumes anwenden lässt und/oder ein Set an unterschiedlichen Typen von modularen Reporterkomplexen unterschiedlich hohe Fluoreszenzsignale durch Mehrfachmarkierungen über z.B. in diesem Fall sehr leicht anzubringende einfache oder mehrfache Signaloligos generieren kann, ohne dass, eine komplexe Synthese notwendig wird. Ohne den Einsatz von aufwendig zu synthetisierenden mehrfachmarkierten Zielsequenz spezifischen Hydrolysesonden wie in der Methode von US 9921154 B2 beschrieben, kann dieses Verfahren nach aktuellem Verständnis nur dann angewendet werden, wenn je Reaktionsraum einer digitalen PCR eine signifikante Mehrfachbelegung mit DNA-Zielsequenzen auftritt, welche so zu unterscheidbaren Signalclustern im Datenraum führen.

Außerdem besitzt ein erfindungsgemäßer Zielsequenz-unabhängiger modularer Reporterkomplex unter PCR-Bedingungen im initialen Zustand eine sehr hohe Stabilität. Das initiale Signal ist entsprechend mit dem vergleichbar, welches durch eine einteilige DNA-Sonde bzw. einem einteiligen Zielsequenz-unabhängigen Reporter generiert wird. Hierdurch besitzt das gesamte resultierende erfindungsgemäße PCR-Nachweis-System vergleichbare Leistungskennzahlen wie aktuelle PCR-Nachweismethoden basierend auf einteiligen Nachweismolekülen. Dieses ist der Fall, obwohl sich dieser Komplex eines Zielsequenz-unabhängigen modularen Reporters mit jedem Zyklus einer PCR aufgetrennt und vor der Signalauslesung erneut gebildet werden muss.

Das erfindungsgemäße System steigert so die Effizienz der Signalgenerierung und ist auch für den gleichzeitigen Nachweis von mehreren DNA-Zielgenen in einer PCR-Reaktion (Multiplex-PCR) geeignet. Zudem bietet es hierbei gänzlich neue Möglichkeiten mehrere DNA-Sequenzen im gleichen Kanal eines PCR Detektors zu detektieren und differenzieren zu können.

In Ausführungsformen des erfindungsgemäßen Verfahrens erfolgen die Schritte c. bis h. im Rahmen einer Reaktion ausgewählt aus der Gruppe umfassend PCR, digitale PCR, RT-PCR, digitale RT-PCR, real-time/qPCR, droplet-PCR, oder jede Kombination dieser.

In einer Ausführungsform des erfindungsgemäßen Verfahrens erfolgen die Schritte c. bis h. im Rahmen einer PCR Reaktion, vorzugsweise einer digitalen PCR Reaktion.

In anderen Ausführungsformen des erfindungsgemäßen Verfahrens erfolgen die Schritte c. bis h. im Rahmen einer PCR Reaktion, vorzugsweise einer real-time PCR oder qPCR Reaktion.

In manchen der zuvor genannten Ausführungsformen des erfindungsgemäßen Verfahrens erfolgen die Schritte c. bis h. im Rahmen einer droplet-PCR oder Emulsions-PCR Reaktion.

Diese unterschiedlichen Anwendungsmöglichkeiten sind möglich, da der erfindungsgemäße Zielsequenz-unabhängige modulare Reporterkomplex zugleich flexibel im Design ist und zum anderen die Einzelkomponenten günstig in ihrer Entwicklung und Produktion sind. Darüber hinaus ermöglicht das erfindungsgemäße System die Herstellung universeller Mikroarrays. Dies stellt insgesamt eine signifikante Verbesserung gegenüber dem Stand der Technik dar.

In einem weiteren Aspekt bezieht sich die Erfindung auf ein Kit für die Durchführung des Verfahrens gemäß einem der vorangehenden Ansprüche umfassend:
- mindestens ein Oligonukleotid-Primer
- mindestens eine Mediatorsonde
- mindestens ein Signaloligo
- mindestens ein Basisstrang
- mindestens ein Puffer
- PCR-Polymerase.

Im Kontext des erfindungsgemäßen Kits können die Oligonukleotid-Primer, vorzugsweise mindestens ein Oligonukleotid-Primer Paar, das mindestens eine Signaloligo, der mindestens eine Mediator und/oder der mindestens eine Basisstrang für die spezifische Detektion eines oder mehrerer unterschiedlicher Zielsequenzen konfiguriert bzw. geeignet sein.

In Ausführungsformen kann das erfindungsgemäße Kit für die Durchführung des erfindungsgemäßen Verfahrens verwendet werden.

In manchen Ausführungsformen kann das Kit somit für die spezifische Amplifikation und/oder Detektion von Zielsequenzen im Rahmen des erfindungsgemäßen Verfahrens verwendet werden. In bevorzugten Ausführungsformen der Verwendung des erfindungsgemäßen Kits ist die spezifische Amplifikations- und/oder Detektionsreaktion eine PCR, qPCR, real-time PCR, droplet PCR and/oder digitale PCR.

Die für einen Aspekt der Erfindung beschriebenen Ausführungsformen können auch Ausführungsformen von jedem der anderen Aspekte der vorliegenden Erfindung sein. Dementsprechend können für das erfindungsgemäße Verfahren beschriebene Ausführungsformen auch Ausführungsformen des erfindungsgemäßen Kits sein. Außerdem kann jede hierin beschriebene Ausführungsform auch Merkmale jeder anderen Ausführungsform der Erfindung umfassen. Die verschiedenen Aspekte der Erfindung werden durch die gemeinsame und überraschende Entdeckung der unerwarteten vorteilhaften Wirkungen des vorliegenden Verfahrens, nämlich der optimierten PCR Detektion von Zielsequenzen durch Reporterkomplexe, welche selbst Zielsequenz unspezifisch sind, vereint, profitieren davon, basieren darauf und/oder sind damit verbunden.

### Detaillierte Beschreibung der Erfindung

Der Begriff "Zielsequenz-unspezifischer Reporterkomplex" beschreibt einen Komplex aus Zielsequenz-unspezifischen Nukleinsäure-Oligonukleotiden (z.B. DNA-Oligonukleotiden) zur Signalgenerierung während einer PCR in Gegenwart von DNA-Zielsequenzen. Vorzugsweise umfasst ein Zielsequenz-unspezifischer Reporterkomplex mindestens eine Markierung, sowie mindestens zwei Oligonukleotide, nämlich 1) einen Basisstrang, umfassend mindestens eine Mediator-Bindestelle und mindestens eine Signaloligo-Bindestelle, und 2) mindestens ein Signaloligo, wobei die Signaloligo-Bindestelle des Basisstrangs und das mindestens eine Signaloligo miteinander hybridisieren, jedoch nicht kovalent verbunden sind.

Im Kontext der vorliegenden Erfindung beschreibt der Begriff "Basisstrang" ein Nukleinsäure-Oligonukleotid (z.B. ein DNA-Oligonukleotid) und Teil des Zielsequenz-unspezifischen Reporterkomplexes. Ein Basisstrang dient als Basis für die Anbindung von Signaloligos und Mediatoren, welche so einen Signalkomplex und Rezeptorkomplex ausbilden. Daher umfasst ein Basisstrang vorzugsweise mindestens eine Mediator-Bindestelle und mindestens eine Signaloligo-Bindestelle. In Ausführungsformen umfasst ein Basisstrang ein oder mehrere Mediator-Bindestellen und/oder Signaloligo-Bindestellen. Somit kann ein Basisstrang mindestens 1, 2, 3, 4, 5, 6, 7, 8, 9, 10, 11, 12, 13, 14, 15, 16, 17, 18, 19, 20, 25, 30, 40 oder sogar 50 Mediator-Bindestellen umfassen. Ein Basisstrang kann zudem mindestens 1, 2, 3, 4, 5, 6, 7, 8, 9, 10, 11, 12, 13, 14, 15, 16, 17, 18, 19, 20, 25, 30, 40 oder sogar 50 Signaloligo-Bindestellen umfassen. In bevorzugten Ausführungsformen umfasst ein Basisstrang zwischen 1 und 10 Mediator-Bindestellen und zwischen 1 und 10 Signaloligo-Bindestellen. Eine Mediator-Bindestelle kann dabei mit einer oder mehreren Signaloligo-Bindestellen korrespondieren, mit anderen Worten, ein an eine Mediator-Bindestelle gebundener (aktivierter) Mediator kann bei seiner Verlängerung durch eine (PCR) Polymerase zurAktivierung bzw. dem Abbau, Verdau, (Ab)Spaltung oder Freisetzung eines oder mehrerer Signaloligos von einer oder mehrerer, vorzugsweise stromaufwärts (gen 5' Ende) gelegenen, Signaloligo-Bindestellen führen. Ein Basisstrang kann eine oder mehrere Signal-Komplexe und ein oder mehrere RezeptorKomplexe umfassen, wobei ein Signaloligo-Komplex mindestens eine Signaloligo-Bindestelle und (im nicht aktivierten Zustand) mindestens ein Signaloligo umfassen kann, und ein Rezeptor-Komplex mindestens eine Mediator-Bindestelle umfassen kann.

Im Kontext der vorliegenden Erfindung ist ein "Signalinitiationsoligonukleotid", "Signalinitiationsoligo" oder kurz "Signaloligo" ein Nukleinsäure-Oligonukleotid (vorzugsweise ein DNA Oligonukleotid) und Teil des Signalkomplexes, welches in Gegenwart einer Zielsequenz eine Signaländerung initiiert, indem es selbst und oder Teile hiervon vom Signalkomplex abgespalten oder aufgebrochen werden. Im Kontext der vorliegenden Erfindung sind die Begriffe Signalinitiationsoligonukleotid, Signalinitiationsoligo und Signaloligo als äquivalent und austauschbar anzusehen.

Im Kontext der vorliegenden Erfindung kann eine "Markierung" ein oder mehrere Fluorophore oder ein oder mehrere Quencher beschreiben. Dementsprechend können im Kontext der Erfindung ein Basisstrang und/oder ein Signalinitiationsmolekül oder Signaloligo in Ausführungsformen ein oder mehrere Fluorophore und/oder Quencher tragen bzw. umfassen. Die Nähe eines Fluorophors zum Quencher verhindert den Nachweis/Detektion seiner Fluoreszenz, wobei der Abbau des Signaloligos durch Hydrolyse durch die 5'-zu-3'-Exonuklease-Aktivität der PCR-Polymerase, die für die Amplifikationsreaktion verwendet wird, die Reporter-Quencher-Nähe unterbricht und somit eine ungelöschte Emission von Fluoreszenz ermöglicht, die nach Anregung mit einem Laser nachgewiesen werden kann. In bevorzugten Ausführungsformen umfasst ein Zielsequenz-unspezifischer modularer Reporter-Komplex mindestens ein Fluorophor und mindestens ein Quencher (diese sind bevorzugt also "paarweise" vorhanden), wobei der Quencher vorzugsweise das Fluorophor-Signal unterdrückt, solange das Signaloligo an der Signaloligo-Bindestelle hybridisiert ist, wobei der mindestens eine Fluorophor und der mindestens eine Quencher zueinander innerhalb des Zielsequenz-unspezifischen modularen Reporter-Komplexes entweder in cis-Stellung (beide entweder am Signaloligo oder am Basisstrang) oder in trans-Stellung (eine der zwei Markierungen ist am Basisstrang, die andere am Signaloligo lokalisiert, oder umgekehrt) lokalisiert sein können. In Ausführungsformen, in denen mehrere Quencher-Fluorophor-Paare innerhalb eines Zielsequenz-unspezifischen modularen Reporter-Komplexes vorhanden sind, können die Paare jeweils auch in unterschiedlichen Lokalisationen zueinander angeordnet sein, z.B. manche in cis- andere in trans-Stellung, oder alle in cis- oder trans-Stellung. Ein Basisstrang kann keine, eine oder mehrere Markierungen umfassen, so können zum Beispiel keine oder mindestens 1, 2, 3, 4, 5, 6, 7, 8, 9, 10, 11, 12, 13, 14, 15, 16, 17, 18, 19, oder 20 Markierungen, oder exakt 0, 1, 2, 3, 4, 5, 6, 7, 8, 9, 10, 11, 12, 13, 14, 15, 16, 17, 18, 19, 20 oder sogar 25 Markierungen vorhanden sein. In manchen Ausführungsformen umfasst ein Basisstrang keine, 1, 2, 3 oder bis zu 5 Markierungen. In anderen Ausführungsformen umfasst ein Basisstrang keine, 1, 2, 3, 4, 5, oder sogar mehr als 5 Markierungen. Ein Signaloligo kann jeweils keine, eine oder mehrere Markierungen umfassen, so können zum Beispiel keine oder mindestens 1, 2, 3, 4, 5, 6, 7, 8, 9, 10, 15, oder sogar 20 Markierungen, oder exakt 0, 1, 2, 3, 4, 5, 6, 7, 8, 9, 10, 11, 12, 13, 14, 15, 16, 17, 18, 19, 20 Markierungen vorhanden sein. In manchen Ausführungsformen umfasst ein Signaloligo keine, 1, 2, 3 oder bis zu 5 Markierungen. In anderen Ausführungsformen umfasst ein Signaloligo keine, 1, 2, 3, 4, 5, oder sogar mehr als 5 Markierungen.

Hierin bezieht sich ein "Mediator" auf ein Oligonukleotid und Teil des Rezeptorkomplexes, welcher durch die Polymerase entlang des "Basisstrangs" verlängert werden kann. Eine "Mediatorsonde" beschreibt ein Nukleinsäure-Oligonukleotid, vorzugsweise ein DNA-Oligonukleotid, welches die Verbindung zwischen der Zielsequenz und dem Zielsequenz-unabhängigen (modularen) Rezeptor herstellt, indem es während einer PCR in Gegenwart einer DNA-Zielsequenz an diese bindet, durch die Exonuklease-Aktivität der Polymerase gespalten wird und eine Mediatorsequenz freisetzt, welche daraufhin an einen Basisstrang bindet und gemeinsam mit dem Basisstrang einen Rezeptor-komplex ausbildet.

Der Begriff "Nukleinsäure" bezieht sich auf Nukleinsäuremoleküle, einschließlich, ohne Einschränkung, DNA, ssDNA, dsDNA, RNA, mRNA, tRNA, IncRNA, ncRNA, microRNA, siRNA, rRNA, sgRNA, piRNA, rmRNA, snRNA, snoRNA, scaRNA, gRNA oder virale RNA. Nukleinsäuresequenzen beziehen sich hierin auf eine aufeinanderfolgende Anordnung von Nukleotiden, wobei die Nukleotide durch ihre Nukleobasen in Guanin (G), Adenin (A), Cytosin (C) und Thymin (T) in DNA und Uracil (U) in RNA dargestellt werden. Eine Nukleinsäuresequenz kann sich hier auch auf die Sequenz aufeinanderfolgender Buchstaben bzw. Nukleobasen (bestehend aus G, A, C und T oder U) beziehen, die die tatsächliche Sequenz aufeinanderfolgender Nukleinsäuren in einem DNA- oder RNA-Strang darstellen. Diese Nukleinsäuresequenz kann unter Verwendung von DNA- oder RNA-Sequenzierung biochemisch und bioinformatisch identifiziert und charakterisiert werden oder durch komplementäre Nukleinsäure-Sonden (z.B. in Ausführungsformen hierin durch Mediatorsonden) spezifisch detektiert werden, z.B. im Rahmen einer PCR, real-time PCR oder Detektionsreaktion einer digitalen PCR. Die Sequenzanalyse kann auch den Vergleich der erhaltenen Nukleinsäuresequenz oder eines dafür spezifischen Detektionssignals mit einer oder mehrerer Referenznukleinsäuresequenzen und/oder mit den Detektionssignalen von Haushaltsgenen umfassen. Der Begriff Nukleotid kann mit "nt" abgekürzt werden. Der Begriff Basenpaar (zwei über Wasserstoffbrücken aneinander gebundene Nukleobasen) kann mit "bp" abgekürzt werden.

Eine "Zielsequenz" beschreibt im Kontext der Erfindung jede Nukleinsäuresequenz von Interesse, welche durch das erfindungsgemäße Verfahren nachgewiesen werden soll. Eine Zielsequenz kann vorzugsweise eine DNA oder RNA Sequenz sein. Eine Zielsequenz kann ein Teil oder die gesamte Nukleinsäuresequenz einer Ziel-DNA darstellen. Eine Mediatorsonde umfasst vorzugsweise eine Sequenz, welche ganz oder teilweise komplementär zu der Nukleinsäuresequenz der Zielsequenz oder einem Teil davon ist. In manchen Ausführungsformen ist diese Sequenz der Mediatorsonde zu 100%, zu 99 %, 95%, 90% oder zu 80% komplementär zu der Zielsequenz. Eine Mediatorsonde kann in manchen Ausführungsformen eine oder mehrere Fehlpaarungen zu der Zielsequenz tolerieren und trotzdem an diese binden. In andere Ausführungsformen bindet die Mediatorsonde nur an eine Zielsequenz, wenn sie zu 100% mit der Zielsequenz übereinstimmt.

Der Begriff "Nukleinsäure-Amplifikationsreaktion" bezieht sich auf jedes Verfahren, das eine enzymatische Reaktion umfasst, die die Amplifikation von Nukleinsäuren ermöglicht. Eine bevorzugte Ausführungsform der Erfindung betrifft eine Polymerase-Kettenreaktion (PCR). Die "Polymerase-Kettenreaktion" ("PCR") ist die Goldstandard Methode, um schnell Millionen bis Milliarden Kopien (vollständige Kopien oder Teilkopien) einer bestimmten DNA-Probe herzustellen, wodurch die Amplifikation einer sehr kleinen DNA-Probe auf eine ausreichend große Menge ermöglicht wird. PCR amplifiziert eine bestimmte Region eines DNA-Strangs (die DNA-Zielsequenz), je nachdem, wo die verwendeten Primer binden, um die Amplifikationsreaktion zu starten. Fast alle PCR-Anwendungen verwenden ein hitzestabiles DNA-Polymerase-Enzym, wie z. B. Taq-Polymerase. Quantitative PCR ("qPCR") oder "real-time PCR" ("Echtzeit-PCR"), stellt eine spezifische Form der PCR dar und ist eine Standardmethode zum Nachweis und zur Quantifizierung einer spezifischen Zielsequenz oder zur Quantifizierung des Genexpressionsniveaus in einer Probe in Echtzeit. Bei der qPCR werden fluoreszenzmarkierte Sonden oder Nukleinsäuren (z.B. Mediator-sonden) in der PCR-Reaktion hybridisiert und in Ausführungsformen von der PCR Polymerase eingebaut bzw. verlängert sobald sie an eine komplementäre Sequenz binden (z.B. eine Zielsequenz), wobei in Ausführungsformen das Vorhandensein und die Amplifikation von Zielsequenzen nach oder während eines jeden PCR-Zyklus in Echtzeit überwacht wird. Eine real-time PCR ermöglicht es, den Fortschritt einer laufenden Amplifikationsreaktion während ihres Auftretens (d. h. in Echtzeit) zu überwachen. Daten werden daher während der exponentiellen Phase der PCR-Reaktion gesammelt und nicht am Endpunkt wie bei der herkömmlichen PCR. Die Messung der Reaktionskinetik in den frühen Phasen der PCR bietet deutliche Vorteile gegenüber dem herkömmlichen PCR-Nachweis. In Ausführungsformen der real-time PCR werden Reaktionen durch den Zeitpunkt während des Zyklus charakterisiert, an dem die Amplifikation eines Ziels erstmals nachgewiesen wird, und nicht durch die Menge des Ziels, die sich nach einer festgelegten Anzahl von Zyklen angesammelt hat, wie bei einer klassischen PCR. Je höher die Startkopienzahl des Nukleinsäure-Targets ist, desto eher wird eine signifikante Zunahme der Fluoreszenz beobachtet. Die real-time PCR ermöglicht eine Analyse mittels optischer Signale, die zum Nachweis eines spezifischen PCR-Produkts (der Zielsequenz), wobei spezifische Fluorochrome bzw. Fluorophore verwendet werden. Eine Zunahme des DNA-Produkts während einer PCR führt daher zu einer Zunahme der bei jedem Zyklus gemessenen Fluoreszenzintensität. Unter Verwendung verschiedenfarbiger Markierungen können fluoreszierende Sonden in Multiplex-Assays zum Überwachen mehrerer Zielsequenzen verwendet werden.

Während die Real-time qPCR davon abhängig ist, dass in jedem Amplifikationszyklus die relative Menge der Zielnukleinsäure bestimmt wird, ermöglicht die "digitale PCR" hingegen eine Bestimmung der absoluten Menge der Zielnukleinsäure auf Basis der Poisson-Statistik, welche im Anschluss an eine Endpunkt-PCR-Amplifikation für die Berechnung der Zielnukleinsäure-Menge angewandt wird. Die Schritte vor der Amplifikation sind üblicherweise vergleichbar oder ähnlich zwischen digitaler PCR und qPCR. Jedoch werden in der qPCR vorzugsweise alle Nukleinsäuremoleküle gepoolt und anschließend amplifiziert und analysiert, während in der digitalen PCR die Nukleinsäuremoleküle vorzugsweise bestmöglich auf Einzelpartitionen (z.B. Emulsions-Tropfen, Kavitäten oder Gel-Beads) aufgeteilt werden, wodurch die PCR in jeder Partition als Einzelreaktion abläuft (im Fall von Emulsions-Tropfen, wird diese Reaktion auch oft als droplet PCR oder digital droplet PCR bezeichnet) und eine separate Analyse jeder Partition ermöglicht. Die zufällige Aufteilung der Nukleinsäuremoleküle in einzelne Partitionen erfolgt bei der digitalen PCR gemäß der Poisson-Verteilung. Bei der Analyse der digitalen PCR wird dann die Poisson-Statistik angewandt, um die durchschnittliche Anzahl an Nukleinsäuremolekülen pro Partition (keines, eines oder mehrere) zu bestimmen. Die statistische Poisson-Analyse der Anzahl positiver und negativer Reaktionen liefert eine präzise absolute Quantifizierung der Target-Sequenz.

Die Spezifität der Mediatorsonden verhindert auch eine Störung der Messungen durch Primer-Dimere, die unerwünschte potenzielle Nebenprodukte bei der PCR sind. In einer Ausführungsform betrifft die Erfindung ein Verfahren, wobei die Amplifikation eine Multiplex-PCR mit mehr als einem Primerpaar ist. Die Multiplex-PCR ist eine Variante der Standard-PCR, bei der zwei oder mehr Zielsequenzen gleichzeitig in derselben Reaktion amplifiziert und/oder nachgewiesen werden können, indem mindestens ein Primerpaar in der Reaktion verwendet wird.

Im Kontext der Erfindung beschreibt eine "Signaländerung" eine Fluoreszenzsignaländerung. Diese Signaländerung ist vorzugsweise eine signifikante, differenzierbare und/oder charakteristische Änderung des Fluoreszenzsignals, welche sich deutlich von potentiellen Basis- oder Hintergrundsignalen bzw. Grund- oder Hintergrundrauschen abgrenzt bzw. unterscheidet. Dem Fachmann ist bewusst, dass unter manchen Versuchsbedingungen im Rahmen von Fluoreszenzdetektionen unspezifische Fluoreszenz-Basissignale bzw. Grund- oder Hintergrundrauschen durch Fluorophore auftreten können. Daher beschreibt eine Signaländerung im Kontext der Erfindung vorzugsweise eine signifikante, differenzierbare und/oder charakteristische Änderung des Fluoreszenzsignals, und kein Fluoreszenz-Basis- oder Hintergrundsignal bzw. Grund- oder Hintergrundrauschen. Diese Signaländerung kann in bevorzugten Ausführungsformen eine Zunahme der Fluoreszenzintensität, in anderen Worten eine Zunahme des Fluoreszenzsignals bedeuten.

In manchen Ausführungsformen ist eine Signaländerung die Abnahme des Fluoreszenzsignals. Die Zunahme eines Fluoreszenzsignals ist vorzugsweise dadurch bedingt, dass durch eine Amplifikationsreaktion die Anzahl der Zielsequenz-Amplifikate steigt und dadurch die Aktivierung zugehöriger Signalkomplexe. Entsprechend steigt die Anzahl der jeweils daraus resultierenden (Ab-)Spaltungen, Verdaue und/oder Trennungen der jeweiligen Signaloligos von ihrer Bindestelle auf den zugehörigen Basissträngen, wodurch je mindestens ein Fluorophor freigesetzt und/oder von seinem Quencher getrennt wird (d.h. der Abstand von Quencher und Fluorophor sich derart vergrößert, dass das Fluoreszenzsignal nicht mehr durch den Quencher gelöscht wird). Eine Zunahme (Vergrößerung der Anzahl) der freigesetzten und/oder nicht-gelöschten Fluorophore führt somit zu einer Zunahme des Fluoreszenzsignals, welches hierin jeweils für eine Zielsequenz spezifisch und indikativ ist. Je mehr Zielsequenzen somit vorhanden sind und durch Mediatorsonden in einer PCR Reaktion gebunden werden, umso mehr steigt das Fluoreszenzsignal. Vorzugsweise ist das Fluoreszenzsignal proportional oder annährend proportional zu der Menge der korrespondierenden Zielsequenz, für welche das Fluorophor-Signal (z.B. seine Farbe) spezifisch/charakteristisch ist. Da im Rahmen einer digitalen oder "droplet" PCR bevorzugt nur eine Zielsequenz je Reaktionsraum (z.B. Partition, Emulsions-Tropfen) vorhanden ist, steigt das Signal zunehmend mit der Anzahl der Zielsequenz-Amplifikate je Reaktionsraum. In bevorzugten Ausführungsformen liegt idealerweise eine gleichmäßige Verteilung von max. 1 Zielsequenz je Reaktionsraum (z.B. Partition, Emulsions-Tropfen) vor, sodass bei beginnender Amplifikation und einer ähnlichen Amplifikationseffizienz in allen Reaktionsräumen (die eine Zielsequenz enthalten) das spezifische Signal für den Nachweis einer identischen Zielsequenz in unterschiedlichen Reaktionsräumen mit vergleichbarer Höhe/Stärke/Intensität bei der Auslese mittels digitaler oder "droplet" PCR entsteht, welches vorzugsweise indikativ für das Vorhandensein und/oder die Anzahl der vorhandenen Zielsequenz-Amplifikate in jedem Reaktionsraum ist. In Ausführungsformen kann die, vorzugsweise für eine Zielsequenz spezifische, Intensität/Stärke einer jeweiligen Markierung sowie die maximal erreichbare Signalstärke/Intensität von der Anzahl der Markierungen je Signaloligo und Signalkomplex und/oder der Art der Markierung (z.B. Art der Fluorophore und/oder Quencher) abhängen.

"Fluorophor" (oder Fluorochrom, ähnlich einem Chromophor) ist eine fluoreszierende chemische Verbindung, die bei Lichtanregung Licht wieder emittieren kann. Fluorophore zur Verwendung als Markierungen bei der Konstruktion markierter Sonden der Erfindung umfassen, ohne Anspruch auf Vollständigkeit zu erheben, Rhodamin und Derivate, wie Texas Red, Fluorescein und Derivate, wie 5-Brommethylfluorescein, LuciferYellow, IAEDANS, 7-Me2N-Cumarin -4-Acetat, 7-OH-4-CH3-Cumarin-3-Acetat, Monobrombiman, Pyrentrisulfonate wie Cascade Blue und Monobromtrimethyl-Ammoniobiman, 7-NH2-4CH3-25-Cumarin-3-Acetat (AMCA), FAM, TET, CAL Fluor Gold 540, JOE, VIC, Quasar 570, CAL Fluor Orange 560, Cy3, NED, Oyster 556, TMR, CAL Fluor Rot 590, HEX, ROX, LC Rot 610, CAL Fluor Rot 610, Texas Rot, LC Rot 610, CAL Fluor Rot 610, LC Rot 640, CAL Fluor Rot 635, Cy5, LC Rot 670, Quasar 670, Oyster 645, LC Rot 705, Cy5.5, BODIPY FL, Rhodamine Green, Oregon Green 30 488, Oregon Green 514, Cal Gold, BODIPY R6Gj, Yakima Yellow, Cal Orange, BODIPY TMR-X, JOE, HEX, Quasar-570 /Cy3, TAMRA, Rhodamine Red-X, Redmond Red, BODIPY 581/591, Cy3.5, Cal Red/Texas Red, BODIPY TR-X , BODIPY 630/665-X, Quasar-670/Cy5, Pulsar-650, Dy490, Atto-488, Atto532, Atto-Rho-6G, Dy590, Atto-Rho101, Cy5, Dy-636, Atto-647N, Cy5.5, Dy682, Atto-680, BMN-488, BMN-505, BMN-536, BMN-562,

"Quenching" bezieht sich auf jeden Prozess, der die Fluoreszenzintensität einer gegebenen Substanz verringert. Quenchen ("Löschen") ist die Grundlage für Förster-Resonanzenergietransfer (FRET)-Assays oder Statische- bzw. Kontaktquenching-Assays oder einer Kombination aus beiden. FRET ist ein dynamischer Löschmechanismus, da die Energieübertragung stattfindet, während sich der Donor im angeregten Zustand befindet. Beim Kontaktquenchen ist eine unmittelbare räumliche Nähe in Form eines physischen Kontakts von Donor und Quencher notwendig. Ein "Quencher" ist ein Molekül, das die vom Fluorophor emittierte Fluoreszenz löscht, wenn dieses durch die Lichtquelle eines PCR-Cyclers oder Detektionsgerätes angeregt wird. Quencher zur Verwendung als Markierungen bei der Konstruktion markierter Signaloligos und/oder Basisstränge der Erfindung umfassen, ohne Anspruch auf Vollständigkeit zu erheben, DDQ-I, Iowa Black, Iowa Black FQ, QSY-9, BHQ-1, QSY-7, BHQ-2, DDQ-II, 22 Eclipse, Iowa Schwarz RQ, QSY-21, BHQ-3 Dabcyl,, QSY-35, BHQ-0, ElleQuencher, BMN-Q1, BMN-Q2, BMN-Q60, BMN-Q-535, BMN-Q590, BMN-Q620, BMN-Q650. Der Fachmann kennt geeignete Reporter-Quencher-Paare und weiß, welche für eine jeweilige Anwendung auszuwählen sind.

### Ausführungsbeispiele

Die Erfindung wird durch die folgenden Beispiele weiter beschrieben. Diese sollen den Umfang der Erfindung nicht einschränken, sondern stellen bevorzugte Ausführungsformen verschiedener Aspekte der Erfindung dar, welche zur Veranschaulichung der hierin beschriebenen Erfindung bereitgestellt werden.

### Zweigeteilter Reporter zur Optimierung von Fluoreszenzsignalen

Der Zielsequenz-unspezifische modulare Reporterkomplex kann in Ausführungsformen aus einem Basisstrang mit einer Markierung am 5'-Ende und einem Signalinitiationsstrang mit einer Markierung am 3'-Ende bestehen (Figur 4B). An den Markierungsstellen werden jeweils ein Fluorophor und ein Quencher angebracht, wodurch im initialen Zustand kein Fluoreszenzsignal generiert wird. Durch Verlängerung des Mediators während der PCR Reaktion durch die PCR-Polymerase, vorzugsweise mit Exonukleaseaktivität, wird die Bindung zwischen Basisstrang und Signalstrang (Signaloligo) aufgebrochen bzw. die Bindung des Fluorophors, sodass ein Signal generiert wird.

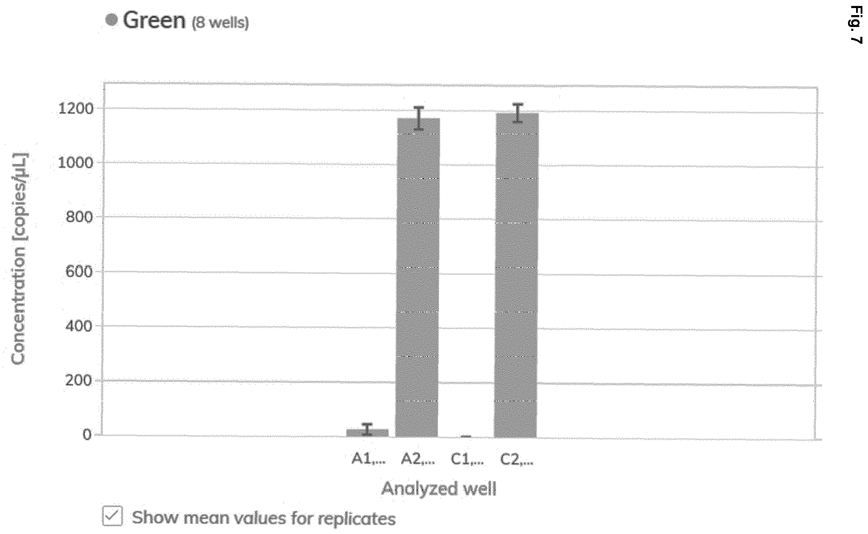

Sowohl die Quenchingeffizienz als auch die Fluoreszenzintensität verschiedener Farbstoffe kann so erleichtert für die PCR-Anwendung optimiert werden, da jeweils nur ein Strang ausgetauscht werden muss, und auch direkt eingesetzt werden kann (Figur 4B). Hierfür ist das System optimal geeignet, da sich das Verhalten und die Leistungsparameter der Modifikationen und Markierungen je nach Sequenz und Anknüpfungsvariante leicht verändern kann. Zudem ist der so eingesetzte Zielsequenz-unspezifische modulare Reporterkomplex wesentlich günstiger und zeigt dabei in der PCR sowie in der digitalen PCR eine gleichgute Leistung wie einteilige universelle Reporter des Standes der Technik. Dieses wurde durch die Ergebnisse von Vergleichsexperimenten belegt, wie die Figuren 6 und 7 zeigen. Durch den Zielsequenz-unabhängigen modularen Reporterkomplex kann demnach die Kombination der Fluorophore und Quencher im Vergleich zu einem einteiligen zweifach markierten Oligonukleotid sehr viel effizienter optimiert werden.

Im vorliegenden Experiment zeigt der zweigeteilte Zielsequenz-unspezifische Reporter zur Optimierung von Fluoreszenzsignalen sowohl im Vergleich zum aktuell, im Stand der Technik eingesetzten, Universal Reporter eine sehr gute Performance als auch im Hinblick auf dessen Verwendung zur Untersuchung von optimalen Fluorophor-Quencher-Kombinationen. In den Ergebnissen eines ersten Vergleichsexperiments, welche in Figur 6 dargestellt sind, wird gezeigt, dass das zweigeteilte System trotz nicht-kovalenter Verbindung zwischen Fluorophor und Quencher, über mehrere Nukleotide des gleichen Moleküls, überraschenderweise hervorragend funktioniert. Wie in Figur 6 zu erkennen ist liegt die Kurve des zweigeteilten modularen Reporters mit einem Quencher-markierten Basisstrang und einem Fluorophor-markierten Signalinitiationsstrang (Figur 6, Kurve mit Kreuzen) und die Kurve des Universal Reporters des Standes der Technik (Figur 6, Kurve mit Dreiecken) exakt aufeinander. Das erfindungsgemäße System ermöglicht nicht nur einen erfolgreichen Nachweis von Zielsequenzen, sondern ermöglicht auch eine gleichzeitige Detektion von mehreren Zielsequenzen. So beweist das Ergebnis, dass mehrere Fluorophore und Quencher verwendet werden können, um das Grundsignal zu variieren und damit z.B. monochrom zu Multiplexen. Dies ist anhand der Kurve des erfindungsgemäßen Reporterkomplexes mit einem Quencher und zwei Fluorophoren (Figur 6, Kurve mit Kreisen, "Multiplexvariante 1") und der Kurve des erfindungsgemäßen Reporterkomplexes mit zwei Quenchern und zwei Fluorophoren (Figur 6, Kurve mit Dreiecken, Multiplexvariante 2") jeweils in Zweifachbestimmung dargestellt.

Ein weiteres überraschendes Ergebnis stellt die optimale Funktionsweise des zweigeteilten Reporters in der digitalen PCR dar. Ein Beispiel dafür ist in dem experimentellen Ergebnis in Figur 7 dargestellt und umfasst einen digitalen PCR Test im grünen Detektionskanal mittels Vergleich des zweigeteilten Reporters (Figur 7, Balken "C1"/"C2") zu einem Universellen Reporter (UR) des Standes der Technik (Figur 7, Balken "A1"/"A2"). Sowohl die negative Amplifizierungskontrolle als auch die positive Amplifizierungskontrolle zeigen, dass das erfindungsgemäße System erfolgreich dieselbe Menge an positiven Tröpfchen nachweisen konnte wie der Stand der Technik-Universelle Reporter (UR).

### Basisstrang mit doppelmarkierten Signalinitiationssträngen

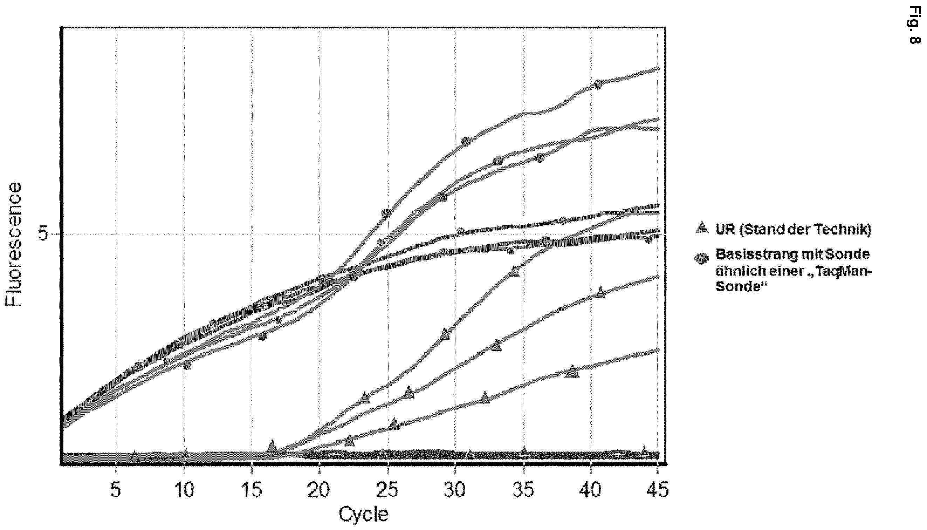

Die Verwendung eines Basisstrangs ohne Markierung bietet ebenfalls Vorteile. Zum einen wird die Herstellung vereinfacht und vergünstigt. Zum anderen können verschiedene Signalinitiationsmoleküle (oder kurz "Signalmoleküle"), welche in diesem Fall ein Fluorophor und Quencher tragen, mit angebunden werden. Beispielsweise können Sondensysteme wie die TaqMan Sonde oder Molecular Beacons an einen Basisstrang angebunden werden, wodurch die Signalgenerierung Zielsequenz-unspezifisch bleibt, aber bereits einfach erhältliche Stand-der-Technik Systeme mit einbindet. Ergebnisse eines Beispielexperiments mit einem Ausführungsbeispiel sind in Figur 8 dargestellt, das vereinfachte Funktionsschema dieses repräsentativen Ausführungsbeispiels findet sich in Figur 4A. Durch Bindung mehrerer doppelmarkierter Signaloligos können z.B. sehr viel stärkere Signaländerungen je Komplex generiert werden als bisher an einteiligen DNA-Sonden oder Reportermolekülen des Standes der Technik. Ein weiteres überraschendes Ergebnis zeigte sich in derVerwendung eines unmarkierten Basisstrangs sowie eines zur"TaqMan Sonde" ähnlichen oder äquivalenten Signaloligos (Figur 8, Kurve mit Kreisen). Es konnte gezeigt werden, dass das erfindungsgemäße modulare System in diesem Fall ein sehr gutes Signal erzeugt und zur Detektion von Zielsequenzen erfolgreich verwendet werden kann. Dies wurde ebenfalls im Vergleich zum Stand der Technik-Universellen Reporter (UR) in drei Kopien (Triplikaten) bewiesen und ist in Figur 8 (Kurve mit Dreiecken) dargestellt.

### Multiplexvariationen

Zum direkten Multiplexen, also dem gleichzeitigen Nachweis verschiedener Zielsequenzen in einer Probe und in einer Reaktion sind nach bisherigem Stand entweder mehrere optische Kanäle, weitere Prozessschritte oder eine aufwendige Konzentrationsabstimmung oder Modifikation der Reportermoleküle notwendig. Im Gegensatz hierzu gibt der erfindungsgemäße Reporter die Möglichkeit, über verschiedene Kanäle kombiniert zu messen, wozu erfindungsgemäße Basisstränge mit mehr als einem Rezeptorkomplex dienen können. In diesen Ausführungsformen sind die Rezeptorkomplexe vorzugsweise so versetzt entlang des Basisstrangs angebracht, dass sie unterschiedliche Signalkomplexe aktivieren (Figur 3). Die möglichen verschiedenen Farb- und/ oder Intensitätskombinationen der jeweiligen Markierungen können hierbei vorzugsweise unterschiedliche verlängerte Mediatorsequenzen und somit unterschiedliche nachgewiesene Zielsequenzen kodieren.

In Ausführungsformen können über verschiedene Fluoreszenzstärken mehrerer Zielsequenzen gleichzeitig nachgewiesen werden, indem verschiedene Zielsequenz-unspezifische modulare Reporterkomplexe eingesetzt werden. Hierbei besitzen diese Zielsequenz-unspezifischen modularen Reporterkomplexe jeweils unterschiedliche Mediatorbindestellen am Basisstrang mit jeweils einer anderen Anzahl an Fluoreszenz- und/ oder Quencher-Markierungen. Verschiedene Ausführungsbeispiele hierfür sind in den Figuren 5A, B und C dargestellt. Auf diese Weise werden von jeden Signal-Komplextyp Signale einer unterschiedlichen Signalstärke erzeugt, welche so in einem Fluoreszenzkanal unterscheidbar werden (siehe die Ergebnisse eines beispielhaften Experimentes in Figur 6). Diese Ausführungsformen bieten insbesondere in der digitalen PCR erhebliche Vorteile um unterschiedliche Signalcluster unterscheidbar zu machen und stellen eine Verbesserung des Standes der Technik dar.

### Figuren

Die Erfindung wird weiter durch die folgenden Figuren beschrieben. Diese sollen den Umfang der Erfindung nicht einschränken, sondern stellen bevorzugte Ausführungsformen von Aspekten der Erfindung dar, die zur Veranschaulichung der hierin beschriebenen Erfindung bereitgestellt werden.
Figur 1: Die Figur zeigt die Mediator-Sonden Spaltung während einer PCR mit Aktivierung des Mediators in einer Ausführungsform der Erfindung.
Figur 2: A)\ zeigt die Signalgenerierung durch Aktivierung des Signaloligos an einem Zielsequenz-unspezifischen modularen universellen Reporterkomplexes in einer Ausführungsform der Erfindung. B) zeigt den schematischen Aufbau einer Ausführungsform eines erfindungsgemäßen Zielsequenz-unspezifischen modularen universellen Reporterkomplexes.
Figur 3: Die Figur zeigt einen Zielsequenz-unspezifischen modularen Reporterkomplex mit alternierenden Signal- und Rezeptorkomplexen, welche z.B. eine Mediator-Signalkodierung für ein monochromes Multiplexing ermöglichen.
Figur 4: Die Figur zeigt Ausführungsbeispiele für verschiedene Markierungspositionen von Fluorophor und Quencher. A) zeigt ein Beispiel für Cis-Markierungen am gleichen Signaloligo. B) zeigt ein Beispiel für Trans-Markierungen.
Figur 5: Die Figur zeigt Ausführungsbeispiele für die Modellierung der Signalstärke (Fluoreszenzsignalstärke) durch Mehrfach-Markierungen eines Zielsequenz-unspezifischen modularen Reporterkomplexes oder Nutzung von unterschiedlichen Quencher-Stärken. A) zeigt ein Beispiel für Cis-Markierungen wobei an einem Basisstrang und dem Signaloligo je ein Quencher und ein Fluorophor vorhanden sind. B) Zeigt ein weiteres Beispiel für Cis-Markierungen, wobei ein Quencher am Basisstrang und zwei Signaloligos mit je einem Fluorophor vorhanden sind. C) zeigt ein Beispiel von Cis-Markierungen, wobei an Basisstrang und Signaloligos je zwei Quencher und zwei Fluorophore vorhanden sind.
Figur 6: Vergleich vom erfindungsgemäßen modularen Reporter zu einem Universellen Reporter (UR) des Standes der Technik (Kurve mit Dreiecken). Die Figur belegt die optimale Funktionsweise des erfindungsgemäßen zweigeteilten Reporters (Kurve mit Kreuzen) im Vergleich zum UR. Die vorteilhaften Eigenschaften des erfindungsgemäßen Reporters bieten auch Möglichkeiten zum Multiplexen von Nachweisreaktionen, z.B. durch die Verwendung von erfindungsgemäßen Reportern mit Markierungen mit verschiedenen Fluoreszenzstärken, z.B. durch einen Quencher und zwei Fluorophore (Multiplexvariante 1, Kurve mit Kreisen) oder durch zwei Quencher und zwei Fluorophore (Multiplexvariante 2, Kurve mit Quadraten). NTCs (engl. Non-template Control) sind jeweils in Dunkelgrau dargestellt.
Figur 7: Darstellung der experimentellen dPCR Ergebnisse einer Ausführungsform des erfindungsgemäßen zweigeteilten Reporters im Vergleich zu einem Stand der Technik UR. Der erfindungsgemäße zweigeteilte Reporter (NTC: C1, PTC: C2) zeigte quantitativ dieselben Ergebnisse wie der UR (NTC: A1, PTC: A2) im Hinblick auf positive und negative Tröpfchen der dPCR.
Figur 8: Vergleich des Universal Reporters (UR) des Standes der Technik (Kurve mit Dreiecken) mit dem erfindungsgemäßen modularen Reporter. Gezeigt ist eine Ausführungsform mit einem nicht-markiertem Basisstrang und einem doppelmarkierten Signaloligo (Kurve mit Kreisen), der einer "TaqMan-Sonde" ähnelt (NTCs sind jeweils in Dunkelgrau dargestellt).

### REFERENZEN

Becherer, Lisa; Bakheit, Mohammed; Frischmann, Sieghard; Stinco, Silvina; Borst, Nadine; Zengerle, Roland; Stetten, Felix von (2018): Simplified Real-Time Multiplex Detection of Loop-Mediated Isothermal Amplification Using Novel Mediator Displacement Probes with Universal Reporters. In: Anal. Chem. 90 (7), S. 4741-4748. DOI: 10.1021/acs.analchem.7b05371.

Faltin, Bernd; Wadle, Simon; Roth, Günter; Zengerle, Roland; Stetten, Felix von (2012): Mediator probe PCR: a novel approach for detection of real-time PCR based on label-free primary probes and standardized secondary universal fluorogenic reporters. In: Clinical Chemistry 58 (11), S. 1546-1556. DOI: 10.1373/clinchem.2012.186734.

Faltin, Bernd; Zengerle, Roland; Stetten, Felix von (2013): Current Methods for Fluorescence-Based Universal Sequence-Dependent Detection of Nucleic Acids in Homogenous Assays and Clinical Applications. In: Clinical Chemistry 59 (11), S. 1567-1582. DOI: 10.1373/clinchem.2013.205211.

Heid, C. A.; Stevens, J.; Livak, K. J.; Williams, P. M. (1996): Real time quantitative PCR. In: Genome Research 6 (10), S. 986-994. DOI: 10.1101/gr.6.10.986.

Holland, P. M.; Abramson, R. D.; Watson, R.; Gelfand, D. H. (1991): Detection of specific polymerase chain reaction product by utilizing the 5'----3' exonuclease activity of Thermus aquaticus DNA polymerase. In: Proceedings of the National Academy of Sciences of the United States of America 88 (16), S. 7276-7280. DOI: 10.1073/pnas.88.16.7276.

Lehnert, Michael; Kipf, Elena; Schlenker, Franziska; Borst, Nadine; Zengerle, Roland; Stetten, Felix von (2018): Fluorescence signal-to-noise optimisation for real-time PCR using universal reporter oligonucleotides. In: Anal. Methods 10 (28), S. 3444-3454. DOI: 10.1039/C8AY00812D.

Li, Yongsheng; Zhou, Xiaoyan; Ye, Duyun (2008): Molecular beacons: an optimal multifunctional biological probe. In: Biochemical and biophysical research communications 373 (4), S. 457-461. DOI: 10.1016/j.bbrc.2008.05.038.

Liu, Shufeng; Fang, Li; Wang, Yanqun; Wang, Li (2017): Universal Dynamic DNA Assembly-Programmed Surface Hybridization Effect for Single-Step, Reusable, and Amplified Electrochemical Nucleic Acid Biosensing. In: Anal. Chem. 89 (5), S. 3108-3115. DOI: 10.1021/acs.anal-chem.6b04871.

Lyamichev, V.; Brow, M. A.; Dahlberg, J. E. (1993): Structure-specific endonucleolytic cleavage of nucleic acids by eubacterial DNA polymerases. In: Science (New York, N.Y.) 260 (5109), S. 778-783. DOI: 10.1126/science.7683443.

Rodriguez, Miguel A.; Garcia, Teresa; Gonzalez, Isabel; Hernändez, Pablo E.; Martin, Rosario (2005): TaqMan real-time PCR for the detection and quantitation of pork in meat mixtures. In: Meat science 70 (1), S. 113-120. DOI: 10.1016/j.meatsci.2004.12.005.

Schlenker, Franziska, Elena Kipf, Max Deuter, Inga Höffkes, Michael Lehnert, Roland Zengerle, Felix von Stetten, Florian Scherer, Julius Wehrle, Nikolas von Bubnoff, Peter Juelg, Tobias Hutzenlaub, and Nadine Borst. 2021. "Stringent Base Specific and Optimization-Free Multiplex Mediator Probe ddPCR for the Quantification of Point Mutations in Circulating Tumor DNA" Cancers 13, no. 22: 5742. https://doi.org/10.3390/cancers13225742

Tan, Weihong; Wang, Kemim; Drake, Timothy J. (2004): Molecular beacons. In: Current opinion in chemical biology 8 (5), S. 547-553. DOI: 10.1016/j.cbpa.2004.08.010.

Tyagi, S.; Kramer, F. R. (1996): Molecular beacons: probes that fluoresce upon hybridization. In: Nat Biotechnol 14 (3), S. 303-308. DOI: 10.1038/nbt0396-303.

Wadle, Simon; Lehnert, Michael; Schuler, Friedrich; Köppel, René; Serr, Annerose; Zengerle, Roland; Stetten, Felix von (2016): Simplified development of multiplex real-time PCR through master mix augmented by universal fluorogenic reporters. In: BioTechniques 61 (3), S. 123-128. DOI: 10.2144/000114443.

Whale, Alexandra S.; Huggett, Jim F.; Tzonev, Svilen (2016): Fundamentals of multiplexing with digital PCR. In: Biomolecular Detection and Quantification 10, S. 15-23. DOI: 10.1016/j.bdq.2016.05.002.

## Patentansprüche

1. Verfahren zum Nachweis mindestens einer Ziel-Nukleinsäuresequenz, umfassend die Schritte:
a. Bereitstellen mindestens eines Zielsequenz-unspezifischen modularen Reporter-Komplexes, umfassend mindestens eine Markierung, sowie mindestens zwei Oligonukleotide, nämlich
i. einen Basisstrang, umfassend
1. mindestens eine Mediator-Bindestelle
2. mindestens eine Signaloligo-Bindestelle
ii. mindestens ein Signaloligo
wobei die mindestens eine Signaloligo-Bindestelle des Basisstrangs und das mindestens eine Signaloligo miteinander hybridisieren, jedoch nicht kovalent verbunden sind und zusammen einen Signal-Komplex bilden,
b. Bereitstellen mindestens einer Mediatorsonde, wobei die Mediatorsonde ein Oligonukleotid mit mindestens einer Sondensequenz und mindestens einer Mediatorsequenz umfasst, wobei
die mindestens eine Sondensequenz eine Affinität zu mindestens einer Ziel-Nukleinsäuresequenz aufweist, und die mindestens eine Mediatorsequenz eine Affinität zu mindestens einer Mediator-Bindestelle an dem Basisstrang des mindestens einen Zielsequenz-unspezifischen modularen Reporter-Komplexes aufweist,
c. PCR Amplifikation mindestens einer Nukleinsäuresequenz,
d. Binden einer Sondensequenz mindestens einer Mediatorsonde an die mindestens eine Ziel-Nukleinsäuresequenz,
e. Spaltung der Sondensequenz der an die mindestens eine Ziel-Nukleinsäuresequenz gebundene mindestens eine Mediatorsonde durch eine PCR Polymerase mit Nukleaseaktivität während der PCR Amplifikation, wobei die Mediatorsequenz freigesetzt wird,
f. Binden mindestens einer freigesetzten Mediatorsequenz an eine Mediator-Bindestelle des mindestens einen Zielsequenz-unspezifischen modularen Reporter-Komplexes,
g. Verlängerung der Sequenz mindestens einer an eine Mediator-Bindestelle gebundenen Mediatorsequenz durch eine PCR Polymerase, wobei die Bindung der miteinander hybridisierten mindestens einen Signaloligo-Bindestelle und des mindestens einen Signaloligos aufgebrochen wird, wodurch eine Signaländerung initiiert wird,
h. Detektion mindestens einer Signaländerung als Nachweis der mindestens einen Ziel-Nukleinsäuresequenz.

2. Verfahren gemäß Anspruch 1, wobei die mindestens eine Markierung mindestens ein Fluorophor und/oder mindestens einen Quencher umfasst.

3. Verfahren gemäß Anspruch 2, wobei keine Fluoreszenzsignaländerung durch den mindestens einen Fluorophor generiert wird, wenn das mindestens eine Signaloligo mit der mindestens einen Signaloligo-Bindestelle des Basisstranges hybridisiert ist,
wobei entweder der mindestens eine Quencher an der mindestens einen Signaloligo-Bindestelle des Basisstranges lokalisiert ist und der mindestens eine Fluorophor an dem mindestens einen Signaloligo oder umgekehrt, und
wobei in Schritt g. mindestens ein Fluorophor und mindestens ein Quencher separiert werden, wodurch eine Signaländerung initiiert wird.

4. Verfahren gemäß Anspruch 2, wobei die mindestens eine Markierung mindestens ein Fluorophor und mindestens ein Quencher umfasst, und
wobei sowohl der mindestens eine Quencher als auch der mindestens eine Fluorophor auf dem mindestens einen Signaloligo lokalisiert sind, und
wobei in Schritt g. das mindestens eine Signaloligo durch die PCR Polymerase abgespalten wird, wodurch der mindestens eine Fluorophor und der mindestens eine Quencher separiert werden, wodurch eine Signaländerung initiiert wird.

5. Verfahren gemäß einem der vorherigen Ansprüche, wobei der Basisstrang mindestens eine Signaloligo-Bindestelle umfasst, an welche zwei oder mehr Signaloligos hybridisiert sind, und wobei die zwei oder mehr Signaloligos und/oder der Basisstrang an der mindestens einen Signaloligo-Bindestelle eine oder mehrere Markierungen besitzen.

6. Verfahren gemäß einem der vorherigen Ansprüche, wobei der Basisstrang zwei oder mehr Signaloligo-Bindestellen umfasst, und wobei mindestens eines der Signaloligos, welche mit den zwei oder mehr Signaloligo-Bindestellen hybridisiert sind und/oder der Basisstrang an mindestens einer der zwei oder mehr Signaloligo-Bindestellen eine oder mehrere Markierungen besitzt.

7. Verfahren gemäß einem der vorherigen Ansprüche, wobei der Basisstrang zwei oder mehr Signaloligo-Bindestellen umfasst, welche zusammen einen Signal-Komplex bilden und an welche jeweils mindestens ein Signaloligo mit jeweils mindestens einer Markierung hybridisiert ist, wobei eine durch die Markierungen des Signal-Komplexes generierte Signaländerung charakteristisch für eine Zielsequenz ist.

8. Verfahren gemäß einem der vorherigen Ansprüche, wobei der Basisstrang mindestens zwei oder mehr Signal-Komplexe mit unterschiedlichen Signaloligos und/oder unterschiedlichen Markierungen an den Signaloligos umfasst, und wobei der Basisstrang einen zu den mindestens zwei oder mehr Signal-Komplexen korrespondierende Mediator-Bindestelle umfasst.

9. Verfahren gemäß einem der vorherigen Ansprüche, wobei der mindestens eine Zielsequenz-unspezifische modulare Reporter-Komplex den Nachweis von mindestens einer ersten und einer zweiten Ziel-Nukleinsäuresequenz ermöglicht, indem der Basisstrang,
mindestens eine erste und eine zweite Mediator-Bindestelle für mindestens eine erste und eine zweite Mediatorsequenz mindestens einer ersten und einer zweiten Mediatorsonde, umfasst, sowie mindestens eine erste und eine zweite Signaloligo-Bindestelle, an welche mindestens ein erstes und ein zweites Signaloligo hybridisiert ist, und
wobei die erste Mediatorsonde eine Sondensequenz mit einer Affinität zu einer ersten Ziel-Nukleinsäuresequenz und die zweite Mediatorsonde eine Sondensequenz mit einer Affinität zu einer zweiten Ziel-Nukleinsäuresequenz aufweist.

10. Verfahren gemäß dem vorhergehenden Anspruch, wobei der Basisstrang mindestens eine erste und eine zweite Markierung aufweist, wobei die Signaländerung durch die mindestens eine erste Markierung charakteristisch für die erste Ziel-Nukleinsäuresequenz ist, und die Signaländerung durch die mindestens eine zweite Markierung charakteristisch für die zweite Ziel-Nukleinsäuresequenz ist.

11. Verfahren gemäß einem der vorherigen Ansprüche, wobei in Schritt a. mindestens ein erster und ein zweiter Zielsequenz-unspezifischer modularer Reporter-Komplex bereitgestellt wird,
wobei der mindestens erste Zielsequenz-unspezifische modulare Reporter-Komplex den Nachweis mindestens einer ersten Ziel-Nukleinsäuresequenz und der mindestens zweite Zielsequenz-unspezifische modulare Reporter-Komplex den Nachweis einer zweiten Ziel-Nukleinsäuresequenz ermöglicht, wobei die Signaländerung durch die mindestens eine Markierung des mindestens ersten Zielsequenz-unspezifischen modularen Reporter-Komplexes charakteristisch für die erste Ziel-Nukleinsäuresequenz und die Signaländerung durch die mindestens eine Markierung des mindestens zweiten Zielsequenz-unspezifischen modularen Reporter-Komplexes charakteristisch für die zweite Ziel-Nukleinsäuresequenz ist.

12. Verfahren gemäß des vorhergehenden Anspruches, wobei sich die für die mindestens erste und die für die mindestens zweite Ziel-Nukleinsäuresequenz charakteristischen Signaländerungen von einander durch ihre Farbe und/oder ihre Fluoreszenz- oder Signalstärke unterscheiden.

13. Verfahren nach einem der vorhergehenden Ansprüche, wobei die Schritte c. bis h. im Rahmen einer Reaktion ausgewählt aus der Gruppe umfassend PCR, digitale PCR, RT-PCR, digitale RT-PCR, real-time/qPCR, droplet PCR, oder jede Kombination dieser, erfolgen.

14. Verfahren gemäß einem der vorherigen Ansprüche, wobei die Detektion der Signaländerung eine Analyse der Signaländerung in Abhängigkeit der Detektions-Temperatur umfasst.

15. Kit für die Durchführung des Verfahrens gemäß einem der vorangehenden Ansprüche umfassend:
- mindestens ein Oligonukleotid-Primer
- mindestens eine Mediatorsonde
- mindestens ein Signaloligo
- mindestens ein Basisstrang
- mindestens ein Puffer
- PCR-Polymerase.
